# EUROPEAN PATENT SPECIFICATION

(11) **EP 1 920 053 B1**
(45) Date of publication and mention of the grant of the patent: **26.10.2011**
(21) Application number: 06761857.9
(22) Date of filing: 15.08.2006
(51) Int. Cl.: C12N 9/54, C12N 15/00, C12N 5/00

(54) **SUBTILASES**
SUBTILASEN
SUBTILASES

(30) Priority: 16.08.2005 DK 200501155; 30.09.2005 DK 200501366
(43) Date of publication of application: 14.05.2008
(73) Proprietor: Novozymes A/S, 2880 Bagsvaerd (DK)
(72) Inventor: NIELSEN, Preben, DK-2970 Hørsholm (DK); BORCHERT, Martin, DK-2100 Copenhagen Oe (DK)
(86) International application number: PCT/DK2006/000446
(87) International publication number: WO 2007/019858

(56) References cited:
- WO-A-03/054185

### SEQUENCE LISTING

<110> Novozymes A/S
<120> Subtilases from Bacillus sp.
<130> 10798.204-WO
<160> 37
<170> PatentIn version 3.3
<210> 1
   <211> 1146
   <212> DNA
   <213> Bacillus sp. strain zi344
<220>
   <221> CDS
   <222> (1) .. (1146)
<400> 1
   atg aat agg aaa gta gga aag tta gtt gca gga ttg gtt tgt gta acc        48
   Met Asn Arg Lys Val Gly Lys Leu Val Ala Gly Leu Val Cys Val Thr
   1               5                   10                  15
   gcc tta tta aca gta aca acc gag gca tct gca gca gaa gaa aaa gta        96
   Ala Leu Leu Thr Val Thr Thr Glu Ala Ser Ala Ala Glu Glu Lys Val
               20                  25                  30
   aaa tat cta atc ggt ttt gaa aaa gaa gct gag ctt gaa gcc ttt gca       144
   Lys Tyr Leu Ile Gly Phe Glu Lys Glu Ala Glu Leu Glu Ala Phe Ala
           35                  40                  45
   aac gag gtt gaa cag gta ggc gtt ttc act aca gat gaa act cag cat       192
   Asn Glu Val Glu Gln Val Gly Val Phe Thr Thr Asp Glu Thr Gln His
       50                  55                  60
   gat gat gag acg att gat gtt gat att att tat gat tat gat tat att       240
   Asp Asp Glu Thr Ile Asp Val Asp Ile Ile Tyr Asp Tyr Asp Tyr Ile
   65                  70                  75                  80
   cca gtc tta tca gta gag att gat cct gag gat gta gaa gca ctt agt       288
   Pro Val Leu Ser Val Glu Ile Asp Pro Glu Asp Val Glu Ala Leu Ser
                   85                  90                  95
   caa gaa gaa ggc att gcc tat att gag gaa gac ttt gaa gta tct att       336
   Gln Glu Glu Gly Ile Ala Tyr Ile Glu Glu Asp Phe Glu Val Ser Ile
               100                 105                 110
   caa cag act gtt cct tgg ggc att caa aga gta caa gct cct gca gtt       384
   Gln Gln Thr Val Pro Trp Gly Ile Gln Arg Val Gln Ala Pro Ala Val
           115                 120                 125
   att aat cgt ggc att aat ggt agc ggg gtg cga gta gcg gtg ctt gat       432
   Ile Asn Arg Gly Ile Asn Gly Ser Gly Val Arg Val Ala Val Leu Asp
       130                 135                 140
   tca ggc att tcc tcc cat agt gat tta agc att tct ggt ggt gta agc      480
   Ser Gly Ile Ser Ser His Ser Asp Leu Ser Ile Ser Gly Gly Val Ser
   145                 150                 155                 160
   ttt gtt cct ggt gaa cca acc ata gcc gat gga aat ggg cac ggg aca      528
   Phe Val Pro Gly Glu Pro Thr Ile Ala Asp Gly Asn Gly His Gly Thr
                   165                 170                 175
   cac gta gct gga acg att gct gca ctt aat aac agc att ggt gtt gta      576
   His Val Ala Gly Thr Ile Ala Ala Leu Asn Asn Ser Ile Gly Val Val
               180                 185                 190
   ggt gtt gca cct aat gct caa att tat gga gta aag gta cta gga gcc      624
   Gly Val Ala Pro Asn Ala Gln Ile Tyr Gly Val Lys Val Leu Gly Ala
           195                 200                 205
   aat ggt cgc gga agt gta agc ggt att gct caa ggt tta gag tgg gct      672
   Asn Gly Arg Gly Ser Val Ser Gly Ile Ala Gln Gly Leu Glu Trp Ala
       210                 215                 220
   gct aca aat aat atg gat att gca aac tta agc cta gga agt gac gcg      720
   Ala Thr Asn Asn Met Asp Ile Ala Asn Leu Ser Leu Gly Ser Asp Ala
   225                 230                 235                 240
   cca agc tca act ctt gaa caa gct gtt aac ttt gcc act agc cga ggt      768
   Pro Ser Ser Thr Leu Glu Gln Ala Val Asn Phe Ala Thr Ser Arg Gly
                   245                 250                 255
   gta ctt gtt gtg gca gct tca gga aat aat gga tct gga aac gtt ggc      816
   Val Leu Val Val Ala Ala Ser Gly Asn Asn Gly Ser Gly Asn Val Gly
               260                 265                 270
   tac cct gca cgt tat gca aat gca atg gcc gtt gga gca aca gat caa      864
   Tyr Pro Ala Arg Tyr Ala Asn Ala Met Ala Val Gly Ala Thr Asp Gln
           275                 280                 285
   aac aat agg cgc gct aac ttt tca caa tat gga gca gga ctt gat att      912
   Asn Asn Arg Arg Ala Asn Phe Ser Gln Tyr Gly Ala Gly Leu Asp Ile
       290                 295                 300
   gta gct cct gga gta ggg gtg caa agt aca tat cct ggt aac cgc tat      960
   Val Ala Pro Gly Val Gly Val Gln Ser Thr Tyr Pro Gly Asn Arg Tyr
   305                 310                 315                 320
   gta agt atg aat gga aca tca atg gca tct cca cac gtt gct ggt gct     1008
   Val Ser Met Asn Gly Thr Ser Met Ala Ser Pro His Val Ala Gly Ala
                   325                 330                 335
   gct gct cta gtg aaa caa aga tat cca tca tgg agt aac act cag att     1056
   Ala Ala Leu Val Lys Gln Arg Tyr Pro Ser Trp Ser Asn Thr Gln Ile
               340                 345                 350
   cgt aat cat ttg aaa aat act gct acg aat ctt gga aac aca aat cag     1104
   Arg Asn His Leu Lys Asn Thr Ala Thr Asn Leu Gly Asn Thr Asn Gln
           355                 360                 365
   ttt ggt agt ggt ctt gta aat gca gac gca gct aca cga taa             1146
   Phe Gly Ser Gly Leu Val Asn Ala Asp Ala Ala Thr Arg
       370                 375                 380
<210> 2
   <211> 381
   <212> PRT
   <213> Bacillus sp. strain Zi344
<400> 2
   Met Asn Arg Lys Val Gly Lys Leu Val Ala Gly Leu Val Cys Val Thr
   1               5                   10                  15
   Ala Leu Leu Thr Val Thr Thr Glu Ala Ser Ala Ala Glu Glu Lys Val
               20                  25                  30
   Lys Tyr Leu Ile Gly Phe Glu Lys Glu Ala Glu Leu Glu Ala Phe Ala
           35                  40                  45
   Asn Glu Val Glu Gln Val Gly Val Phe Thr Thr Asp Glu Thr Gln His
       50                  55                  60
   Asp Asp Glu Thr Ile Asp Val Asp Ile Ile Tyr Asp Tyr Asp Tyr Ile
   65                  70                  75                  80
   Pro Val Leu Ser Val Glu Ile Asp Pro Glu Asp Val Glu Ala Leu Ser
                   85                  90                  95
   Gln Glu Glu Gly Ile Ala Tyr Ile Glu Glu Asp Phe Glu Val Ser Ile
               100                 105                 110
   Gln Gln Thr Val Pro Trp Gly Ile Gln Arg Val Gln Ala Pro Ala Val
           115                 120                 125
   Ile Asn Arg Gly Ile Asn Gly Ser Gly Val Arg Val Ala Val Leu Asp
       130                 135                 140
   Ser Gly Ile Ser Ser His Ser Asp Leu Ser Ile Ser Gly Gly Val Ser
   145                 150                 155                 160
   Phe Val Pro Gly Glu Pro Thr Ile Ala Asp Gly Asn Gly His Gly Thr
                   165                 170                 175
   His Val Ala Gly Thr Ile Ala Ala Leu Asn Asn Ser Ile Gly Val Val
               180                 185                 190
   Gly Val Ala Pro Asn Ala Gln Ile Tyr Gly Val Lys Val Leu Gly Ala
           195                 200                 205
   Asn Gly Arg Gly Ser Val Ser Gly Ile Ala Gln Gly Leu Glu Trp Ala
   210 215 220
   Ala Thr Asn Asn Met Asp Ile Ala Asn Leu Ser Leu Gly Ser Asp Ala
   225 230 235 240
   Pro Ser Ser Thr Leu Glu Gln Ala Val Asn Phe Ala Thr Ser Arg Gly
   245 250 255
   Val Leu Val Val Ala Ala Ser Gly Asn Asn Gly Ser Gly Asn Val Gly
   260 265 270
   Tyr Pro Ala Arg Tyr Ala Asn Ala Met Ala Val Gly Ala Thr Asp Gln
   275 280 285
   Asn Asn Arg Arg Ala Asn Phe Ser Gln Tyr Gly Ala Gly Leu Asp Ile
   290 295 300
   Val Ala Pro Gly Val Gly Val Gln Ser Thr Tyr Pro Gly Asn Arg Tyr
   305 310 315 320
   Val Ser Met Asn Gly Thr Ser Met Ala Ser Pro His Val Ala Gly Ala
   325 330 335
   Ala Ala Leu Val Lys Gln Arg Tyr Pro Ser Trp Ser Asn Thr Gln Ile
   340 345 350
   Arg Asn His Leu Lys Asn Thr Ala Thr Asn Leu Gly Asn Thr Asn Gln
   355 360 365
   Phe Gly Ser Gly Leu Val Asn Ala Asp Ala Ala Thr Arg
   370 375 380
<210> 3
   <211> 1152
   <212> DNA
   <213> Bacillus sp. strain EP655
<220>
   <221> CDS
   <222> (1)..(1152)
<400> 3
   atg aaa aga aag att gga aaa ctt gtt gta gga ctt gtt tgt gta aca 48
   Met Lys Arg Lys Ile Gly Lys Leu Val Val Gly Leu Val Cys Val Thr
   1               5                   10                  15
   gcc ctt gtt agt gtg aca gac tca gca tca gct gca gaa gaa aag gta      96
   Ala Leu Val Ser Val Thr Asp Ser Ala Ser Ala Ala Glu Glu Lys Val
               20                  25                  30
   aag tac cta att ggt ttt gaa aaa gaa gct gaa ctt gaa gct ttt aca     144
   Lys Tyr Leu Ile Gly Phe Glu Lys Glu Ala Glu Leu Glu Ala Phe Thr
           35                  40                  45
   gat gaa gtt gag cag gtt ggc gta ttc tct att gaa gaa gat cag caa     192
   Asp Glu Val Glu Gln Val Gly Val Phe Ser Ile Glu Glu Asp Gln Gln
       50                  55                  60
   aaa gaa gat tcg act gat att gat gta gac att att ttt gat tac gat     240
   Lys Glu Asp Ser Thr Asp Ile Asp Val Asp Ile Ile Phe Asp Tyr Asp
   65                  70                  75                  80
   tat att ccc gta tta tca gtt gag ttg gac cct gaa gat gtt gat gca     288
   Tyr Ile Pro Val Leu Ser Val Glu Leu Asp Pro Glu Asp Val Asp Ala
                   85                  90                  95
   tta agt gaa gaa gat gga atc gca tat att gaa gaa gac ttt gaa gta     336
   Leu Ser Glu Glu Asp Gly Ile Ala Tyr Ile Glu Glu Asp Phe Glu Val
               100                 105                 110
   tca atc cag caa tcg gtg cct tgg ggt att act cgt gta caa gct cca     384
   Ser Ile Gln Gln Ser Val Pro Trp Gly Ile Thr Arg Val Gln Ala Pro
           115                 120                 125
   gca gcg att aac cgt gga aca aat ggt tca gga gta aga gcg gct gta     432
   Ala Ala Ile Asn Arg Gly Thr Asn Gly Ser Gly Val Arg Ala Ala Val
       130                 135                 140
   ttg gat aca gga att tct aca cat agt gat tta aca att cgt ggt gga     480
   Leu Asp Thr Gly Ile Ser Thr His Ser Asp Leu Thr Ile Arg Gly Gly
   145                 150                 155                 160
   gct agc ttc gtg cct ggt gaa cca aat aca tct gac tta aat ggc cat     528
   Ala Ser Phe Val Pro Gly Glu Pro Asn Thr Ser Asp Leu Asn Gly His
                   165                 170                 175
   ggt acc cat gta gct gga aca att gca gct ttg aat aac tca atc ggc     576
   Gly Thr His Val Ala Gly Thr Ile Ala Ala Leu Asn Asn Ser Ile Gly
               180                 185                 190
   gtt gta ggt gta gca cca aat gct gat cta tat gct gta aaa gtt ctt     624
   Val Val Gly Val Ala Pro Asn Ala Asp Leu Tyr Ala Val Lys Val Leu
           195                 200                 205
   ggg gca aat ggt aga gga agc att gga gga att gca caa ggt tta gag     672
   Gly Ala Asn Gly Arg Gly Ser Ile Gly Gly Ile Ala Gln Gly Leu Glu
       210                 215                 220
   tgg gca gct gcg aac aat atg cac ata gca aac ttg agc ctt ggt agc     720
   Trp Ala Ala Ala Asn Asn Met His Ile Ala Asn Leu Ser Leu Gly Ser
   225                 230                 235                 240
   gat gca cct agc tca act ctt gag cag gct gtt aat tac gct aca agt     768
   Asp Ala Pro Ser Ser Thr Leu Glu Gln Ala Val Asn Tyr Ala Thr Ser
                   245                 250                 255
   cgc ggt gta tta gtt att gcg gct tca ggt aat aac ggt tca ggt aac       816
   Arg Gly Val Leu Val Ile Ala Ala Ser Gly Asn Asn Gly Ser Gly Asn
               260                 265                 270
   gtt gga tat cct gca cgt tat gct aat gca atg gca gta gga gca acc       864
   Val Gly Tyr Pro Ala Arg Tyr Ala Asn Ala Met Ala Val Gly Ala Thr
           275                 280                 285
   gat caa aat aat aac cgt gct aac ttc tct caa tat ggt gca gga ctt       912
   Asp Gln Asn Asn Asn Arg Ala Asn Phe Ser Gln Tyr Gly Ala Gly Leu
       290                 295                 300
   gat atc gta gct cca ggt gta ggc att caa agt acg tat cct ggt aac       960
   Asp Ile Val Ala Pro Gly Val Gly Ile Gln Ser Thr Tyr Pro Gly Asn
   305                 310                 315                 320
   cgc tat gcg agc cta aat ggt aca tct atg gca act cct cac gtt gca      1008
   Arg Tyr Ala Ser Leu Asn Gly Thr Ser Met Ala Thr Pro His Val Ala
                   325                 330                 335
   gga gcg gca gca ctt gta aaa caa cgc tat cct tct tgg agt gca tcg      1056
   Gly Ala Ala Ala Leu Val Lys Gln Arg Tyr Pro Ser Trp Ser Ala Ser
               340                 345                 350
   caa atc cgt aat cat ctg aaa aac aca tct acg aat cta gga agc tct      1104
   Gln Ile Arg Asn His Leu Lys Asn Thr Ser Thr Asn Leu Gly Ser Ser
           355                 360                 365
   aca tta tat ggt agt gga tta gta aac gca gat gcc gct agt cga taa      1152
   Thr Leu Tyr Gly Ser Gly Leu Val Asn Ala Asp Ala Ala Ser Arg
       370                 375                 380
<210> 4
   <211> 383
   <212> PRT
   <213> Bacillus sp. strain EP655
<400> 4
   Met Lys Arg Lys Ile Gly Lys Leu Val Val Gly Leu Val Cys Val Thr
   1               5                   10                  15
   Ala Leu Val Ser Val Thr Asp Ser Ala Ser Ala Ala Glu Glu Lys Val
               20                  25                  30
   Lys Tyr Leu Ile Gly Phe Glu Lys Glu Ala Glu Leu Glu Ala Phe Thr
           35                  40                  45
   Asp Glu Val Glu Gln Val Gly Val Phe Ser Ile Glu Glu Asp Gln Gln
       50                  55                  60
   Lys Glu Asp Ser Thr Asp Ile Asp Val Asp Ile Ile Phe Asp Tyr Asp
   65                  70                  75                  80
   Tyr Ile Pro Val Leu Ser Val Glu Leu Asp Pro Glu Asp Val Asp Ala
                   85                  90                  95
   Leu Ser Glu Glu Asp Gly Ile Ala Tyr Ile Glu Glu Asp Phe Glu Val
               100                 105                 110
   Ser Ile Gln Gln Ser Val Pro Trp Gly Ile Thr Arg Val Gln Ala Pro
           115                 120                 125
   Ala Ala Ile Asn Arg Gly Thr Asn Gly Ser Gly Val Arg Ala Ala Val
       130                 135                 140
   Leu Asp Thr Gly Ile Ser Thr His Ser Asp Leu Thr Ile Arg Gly Gly
   145                 150                 155                 160
   Ala Ser Phe Val Pro Gly Glu Pro Asn Thr Ser Asp Leu Asn Gly His
                   165                 170                 175
   Gly Thr His Val Ala Gly Thr Ile Ala Ala Leu Asn Asn Ser Ile Gly
               180                 185                 190
   Val Val Gly Val Ala Pro Asn Ala Asp Leu Tyr Ala Val Lys Val Leu
           195                 200                 205
   Gly Ala Asn Gly Arg Gly Ser Ile Gly Gly Ile Ala Gln Gly Leu Glu
       210                 215                 220
   Trp Ala Ala Ala Asn Asn Met His Ile Ala Asn Leu Ser Leu Gly Ser
   225                 230                 235                 240
   Asp Ala Pro Ser Ser Thr Leu Glu Gln Ala Val Asn Tyr Ala Thr Ser
                   245                 250                 255
   Arg Gly Val Leu Val Ile Ala Ala Ser Gly Asn Asn Gly Ser Gly Asn
               260                 265                 270
   Val Gly Tyr Pro Ala Arg Tyr Ala Asn Ala Met Ala Val Gly Ala Thr
           275                 280                 285
   Asp Gln Asn Asn Asn Arg Ala Asn Phe Ser Gln Tyr Gly Ala Gly Leu
       290                 295                 300
   Asp Ile Val Ala Pro Gly Val Gly Ile Gln Ser Thr Tyr Pro Gly Asn
   305                 310                 315                 320
   Arg Tyr Ala Ser Leu Asn Gly Thr Ser Met Ala Thr Pro His Val Ala
                   325                 330                 335
   Gly Ala Ala Ala Leu Val Lys Gln Arg Tyr Pro Ser Trp Ser Ala Ser
               340                 345                 350
   Gln Ile Arg Asn His Leu Lys Asn Thr Ser Thr Asn Leu Gly Ser Ser
           355                 360                 365
   Thr Leu Tyr Gly Ser Gly Leu Val Asn Ala Asp Ala Ala Ser Arg
       370                 375                 380
<210> 5
   <211> 1152
   <212> DNA
   <213> Bacillus sp. strain p203
<220>
   <221> CDS
   <222> (1)..(1152)
<400> 5
   atg aaa aga aag att gga aaa ctt gtt gta gga ctt gtt tgt gta aca      48
   Met Lys Arg Lys Ile Gly Lys Leu Val Val Gly Leu Val Cys Val Thr
   1               5                   10                  15
   gcc ctt gtt agt gtg aca gac tca gca tca gct gca gaa gaa aag gta      96
   Ala Leu Val Ser Val Thr Asp Ser Ala Ser Ala Ala Glu Glu Lys Val
               20                  25                  30
   aag tac cta att ggt ttt gaa aaa gaa gct gaa ctt gaa gct ttt aca     144
   Lys Tyr Leu Ile Gly Phe Glu Lys Glu Ala Glu Leu Glu Ala Phe Thr
           35                  40                  45
   gat gaa gtt gag cag gtt ggc gta ttc tct att gaa gaa gat cag caa     192
   Asp Glu Val Glu Gln Val Gly Val Phe Ser Ile Glu Glu Asp Gln Gln
       50                  55                  60
   aaa gaa gat tcg act gat att gat gta gac att att ttt gat tac gat     240
   Lys Glu Asp Ser Thr Asp Ile Asp Val Asp Ile Ile Phe Asp Tyr Asp
   65                  70                  75                  80
   tat att ccc gta tta tca gtt gag ttg gac cct gaa gat gtt gat gca     288
   Tyr Ile Pro Val Leu Ser Val Glu Leu Asp Pro Glu Asp Val Asp Ala
                   85                  90                  95
   tta agt gaa gaa gat gga atc gca tat att gaa gaa gac ttt gag gta     336
   Leu Ser Glu Glu Asp Gly Ile Ala Tyr Ile Glu Glu Asp Phe Glu Val
               100                 105                 110
   tca atc cag caa tcg gtg cct tgg ggt att act cgt gta caa gct cca      384
   Ser Ile Gln Gln Ser Val Pro Trp Gly Ile Thr Arg Val Gln Ala Pro
           115                 120                 125
   gca gcg att aac cgt gga aca aat ggt tca gga gta aga gtg gct gta      432
   Ala Ala Ile Asn Arg Gly Thr Asn Gly Ser Gly Val Arg Val Ala Val
       130                 135                 140
   ttg gat aca gga att tct aca cat agt gat tta aca att cgt ggt gga      480
   Leu Asp Thr Gly Ile Ser Thr His Ser Asp Leu Thr Ile Arg Gly Gly
   145                 150                 155                 160
   gct agc ttc gtg cct ggt gaa cca aat aca tct gac tta aat ggc cat      528
   Ala Ser Phe Val Pro Gly Glu Pro Asn Thr Ser Asp Leu Asn Gly His
                   165                 170                 175
   ggt acc cat gta gct gga aca att gca gct ttg aat aac tca atc ggc      576
   Gly Thr His Val Ala Gly Thr Ile Ala Ala Leu Asn Asn Ser Ile Gly
               180                 185                 190
   gtt gta ggt gta gca cca aat gct gat cta tat gct gta aaa gtt ctt      624
   Val Val Gly Val Ala Pro Asn Ala Asp Leu Tyr Ala Val Lys Val Leu
           195                 200                 205
   ggg gca aat ggt aga gga agc att gga gga att gca caa ggt tta ggg      672
   Gly Ala Asn Gly Arg Gly Ser Ile Gly Gly Ile Ala Gln Gly Leu Gly
       210                 215                 220
   tgg gca gct gcg aac aat atg cac ata gca aac ttg agc ctt ggt agc      720
   Trp Ala Ala Ala Asn Asn Met His Ile Ala Asn Leu Ser Leu Gly Ser
   225                 230                 235                 240
   gat gca cct agc tca act ctt gag cag gct gtt aat tac gct aca agt      768
   Asp Ala Pro Ser Ser Thr Leu Glu Gln Ala Val Asn Tyr Ala Thr Ser
                   245                 250                 255
   cgc ggt gta tta gtt att gcg gct tca ggt aat aac ggt tca ggt aac      816
   Arg Gly Val Leu Val Ile Ala Ala Ser Gly Asn Asn Gly Ser Gly Asn
               260                 265                 270
   gtt gga tat cct gca cgt tat gct aat gca atg gca gta gga gca acc      864
   Val Gly Tyr Pro Ala Arg Tyr Ala Asn Ala Met Ala Val Gly Ala Thr
           275                 280                 285
   gat caa aat aat aac cgt gct aac ttc tct caa tat ggt gca gga ctt      912
   Asp Gln Asn Asn Asn Arg Ala Asn Phe Ser Gln Tyr Gly Ala Gly Leu
       290                 295                 300
   gat atc gta gct cca ggt gta ggc att caa agt acg tat cct ggt aac      960
   Asp Ile Val Ala Pro Gly Val Gly Ile Gln Ser Thr Tyr Pro Gly Asn
   305                 310                 315                 320
   cgc tat gcg agc cta aat ggt aca tct atg gca act cct cac gtt gca     1008
   Arg Tyr Ala Ser Leu Asn Gly Thr Ser Met Ala Thr Pro His Val Ala
                   325                 330                 335
   gga gcg gca gca ctt gta aaa caa cgc tat cct tct tgg agt gca tcg     1056
   Gly Ala Ala Ala Leu Val Lys Gln Arg Tyr Pro Ser Trp Ser Ala Ser
   340 345 350
   caa atc cgt aat cat ctg aaa aac aca tct acg aat cta gga agc tct 1104
   Gln Ile Arg Asn His Leu Lys Asn Thr Ser Thr Asn Leu Gly Ser Ser
   355 360 365
   aca tta tat ggt agt gga tta gta aac gca gat gcc gct agt cga taa 1152
   Thr Leu Tyr Gly Ser Gly Leu Val Asn Ala Asp Ala Ala Ser Arg
   370 375 380
<210> 6
   <211> 383
   <212> PRT
   <213> Bacillus sp. strain p203
<400> 6
   Met Lys Arg Lys Ile Gly Lys Leu Val Val Gly Leu Val Cys Val Thr
   1 5 10 15
   Ala Leu Val Ser Val Thr Asp Ser Ala Ser Ala Ala Glu Glu Lys Val
   20 25 30
   Lys Tyr Leu Ile Gly Phe Glu Lys Glu Ala Glu Leu Glu Ala Phe Thr
   35 40 45
   Asp Glu Val Glu Gln Val Gly Val Phe Ser Ile Glu Glu Asp Gln Gln
   50 55 60
   Lys Glu Asp Ser Thr Asp Ile Asp Val Asp Ile Ile Phe Asp Tyr Asp
   65 70 75 80
   Tyr Ile Pro Val Leu Ser Val Glu Leu Asp Pro Glu Asp Val Asp Ala
   85 90 95
   Leu Ser Glu Glu Asp Gly Ile Ala Tyr Ile Glu Glu Asp Phe Glu Val
   100 105 110
   Ser Ile Gln Gln Ser Val Pro Trp Gly Ile Thr Arg Val Gln Ala Pro
   115 120 125
   Ala Ala Ile Asn Arg Gly Thr Asn Gly Ser Gly Val Arg Val Ala Val
   130 135 140
   Leu Asp Thr Gly Ile Ser Thr His Ser Asp Leu Thr Ile Arg Gly Gly
   145 150 155 160
   Ala Ser Phe Val Pro Gly Glu Pro Asn Thr Ser Asp Leu Asn Gly His
   165 170 175
   Gly Thr His Val Ala Gly Thr Ile Ala Ala Leu Asn Asn Ser Ile Gly
   180 185 190
   Val Val Gly Val Ala Pro Asn Ala Asp Leu Tyr Ala Val Lys Val Leu
   195 200 205
   Gly Ala Asn Gly Arg Gly Ser Ile Gly Gly Ile Ala Gln Gly Leu Gly
   210 215 220
   Trp Ala Ala Ala Asn Asn Met His Ile Ala Asn Leu Ser Leu Gly Ser
   225 230 235 240
   Asp Ala Pro Ser Ser Thr Leu Glu Gln Ala Val Asn Tyr Ala Thr Ser
   245 250 255
   Arg Gly Val Leu Val Ile Ala Ala Ser Gly Asn Asn Gly Ser Gly Asn
   260 265 270
   Val Gly Tyr Pro Ala Arg Tyr Ala Asn Ala Met Ala Val Gly Ala Thr
   275 280 285
   Asp Gln Asn Asn Asn Arg Ala Asn Phe Ser Gln Tyr Gly Ala Gly Leu
   290 295 300
   Asp Ile Val Ala Pro Gly Val Gly Ile Gln Ser Thr Tyr Pro Gly Asn
   305 310 315 320
   Arg Tyr Ala Ser Leu Asn Gly Thr Ser Met Ala Thr Pro His Val Ala
   325 330 335
   Gly Ala Ala Ala Leu Val Lys Gln Arg Tyr Pro Ser Trp Ser Ala Ser
   340 345 350
   Gln Ile Arg Asn His Leu Lys Asn Thr Ser Thr Asn Leu Gly Ser Ser
   355 360 365
   Thr Leu Tyr Gly Ser Gly Leu Val Asn Ala Asp Ala Ala Ser Arg
   370 375 380
<210> 7
   <211> 20
   <212> DNA
   <213> Artificial
<220>
   <223> Primer
<220>
   <221> misc_feature
   <222> (9) .. (9)
   <223> n in position 9 is inosine
<220>
   <221> misc_feature
   <222> (15) .. (15)
   <223> n in position 15 is inosine
<220>
   <221> misc_feature
   <222> (18) .. (18)
   <223> r in position 18 is a 50/50 mixture of A and G
<400> 7
   gaggaggcng agttngargc 20
<210> 8
   <211> 24
   <212> DNA
   <213> Artificial
<220>
   <223> Primer
<220>
   <221> misc feature
   <222> (1) .. (24)
<400> 8
   agttagcaga tataaataat tcaa 24
<210> 9
   <211> 23
   <212> DNA
   <213> Artificial
<220>
   <223> Primer
<220>
   <221> misc_feature
   <222> (1) .. (23)
<400> 9
   gtggagtagc catagatgta cca 23
<210> 10
   <211> 20
   <212> DNA
   <213> Artificial
<220>
   <223> Primer
<220>
   <221> misc_feature
   <222> (1)..(20)
<400> 10
   tgcaaacgag gttgaacagg 20
<210> 11
   <211> 20
   <212> DNA
   <213> Artificial
<220>
   <223> Primer
<220>
   <221> misc_feature
   <222> (1) .. (20)
<400> 11
   acacgagtaa taccccaagg 20
<210> 12
   <211> 20
   <212> DNA
   <213> Artificial
<220>
   <223> Primer
<220>
   <221> misc_feature
   <222> (1) .. (20)
<400> 12
   gctaatgcaa tggcagtagg 20
<210> 13
   <211> 20
   <212> DNA
   <213> Artificial
<220>
   <223> Primer
<220>
   <221> misc_feature
   <222> (1) .. (20)
   <400> 13
actctttgaa tgccccaagg 20
<210> 14
   <211> 20
   <212> DNA
   <213> Artificial
<220>
   <223> Primer
<220>
   <221> misc_feature
   <222> (1) .. (20)
<400> 14
   aggtgtactt gttgtggcag 20
<210> 15
   <211> 20
   <212> DNA
   <213> Artificial
<220>
   <223> Primer
<220>
   <221> misc_feature
   <222> (1) .. (20)
<400> 15
   agtaataccc caaggcaccg 20
<210> 16
   <211> 20
   <212> DNA
   <213> Artificial
<220>
   <223> Primer
<220>
   <221> misc_feature
   <222> (1) .. (20)
<400> 16
   gcggcttcag gtaataacgg 20
<210> 17
   <211> 20
   <212> DNA
   <213> Artificial
<220>
   <223> Primer
<220>
   <221> misc_feature
   <222> (1) .. (20)
<400> 17
   caactcaact gataatacgg 20
<210> 18
   <211> 20
   <212> DNA
   <213> Artificial
<220>
   <223> Primer
<220>
   <221> misc_feature
   <222> (1).. (20)
<400> 18
   ttctctcaat atggtgcagg 20
<210> 19
   <211> 20
   <212> DNA
   <213> Artificial
<220>
   <223> Primer
<220>
   <221> misc_feature
   <222> (1)..(20)
<400> 19
   aatgcatcaa catcttcagg 20
<210> 20
   <211> 20
   <212> DNA
   <213> Artificial
<220>
   <223> Primer
<220>
   <221> misc_feature
   <222> (1) .. (20)
<400> 20
   ggatatcctg cacgttatgc 20
<210> 21
   <211> 20
   <212> DNA
   <213> Artificial
<220>
   <223> Primer
<220> .
   <221> misc_feature
   <222> (1) .. (20)
<400> 21
   agtgcttcta catcctcagg 20
<210> 22
   <211> 20
   <212> DNA
   <213> Artificial
<220>
   <223> Primer
<220>
   <221> misc feature
   <222> (1) .. (20)
<400> 22
   aacgttggct accctgcacg 20
<210> 23
   <211> 31
   <212> DNA
   <213> Artificial
<220>
   <223> Primer
<220>
   <221> misc_feature
   <222> (1) .. (31)
<400> 23
   ttatggagct cctaaaaatg aggaggcgac c 31
<210> 24
   <211> 31
   <212> DNA
   <213> Artificial
<220>
   <223> Primer
<220>
   <221> misc feature
   <222> (1) .. (31)
<400> 24
   tgtatggatc caaatagaga cgaaaccgcc c 31
<210> 25
   <211> 31
   <212> DNA
   <213> Artificial
<220>
   <223> Primer
   t
<220>
   <221> misc_feature
   <222> (1)..(31)
<400> 25
   gattaacgcg tctgctctta tcgactagcg g 31
<210> 26
   <211> 31
   <212> DNA
   <213> Artificial
<220>
   <223> Primer
<220>
   <221> misc_feature
   <222> (1) .. (31)
<400> 26
   ttatggagct cgatcaatac aaggaggcga c 31
<210> 27
   <211> 31
   <212> DNA
   <213> Artificial
<220>
   <223> Primer
<220>
   <221> misc_feature
   <222> (1) .. (31)
<400> 27
   gattaacgcg tgttctttta tcgtgtagct g 31
<210> 28
   <211> 828
   <212> DNA
   <213> Bacillus sp. strain EP63
<220>
   <221> CDS
   <222> (199)..(828)
<400> 28
<210> 29
   <211> 210
   <212> PRT
   <213> Bacillus sp. strain EP63
<400> 29
   Gln Gln Ser Val Pro Trp Gly Ile Thr Arg Val Gln Ala Pro Ala Ala
   1               5                   10                  15
   Ile Asn Arg Gly Thr Asn Gly Ser Gly Val Arg Val Ala Val Leu Asp
               20                  25                  30
   Thr Gly Ile Ser Thr His Ser Asp Leu Thr Ile Arg Gly Gly Ala Ser
           35                  40                  45
   Phe Val Pro Gly Glu Pro Asn Thr Ser Asp Leu Asn Gly His Gly Thr
       50                  55                  60
   His Val Ala Gly Thr Ile Ala Ala Leu Asn Asn Ser Ile Gly Val Val
   65                  70                  75                  80
   Gly Val Ala Pro Asn Ala Asp Leu Tyr Ala Val Lys Val Leu Gly Ala
                   85                  90                  95
   Asn Gly Arg Gly Ser Ile Gly Gly Ile Ala Gln Gly Leu Glu Trp Ala
               100                 105                 110
   Ala Ala Asn Asn Met His Ile Ala Asn Leu Ser Leu Gly Ser Asp Ala
           115                 120                 125
   Pro Ser Ser Thr Leu Glu Gln Ala Val Asn Tyr Ala Thr Ser Arg Gly
       130                 135                 140
   Val Leu Val Ile Ala Ala Ser Gly Asn Asn Gly Ser Gly Asn Val Gly
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Asn Phe Ser Gln Tyr Gly Ala Gly Leu Asp Ile
               180                 185                 190
   Val Ala Pro Gly Val Gly Ile Gln Ser Thr Tyr Pro Gly Asn Arg Tyr
           195                 200                 205
   Ala Ser
       210
<210> 30
   <211> 834
   <212> DNA
   <213> Bacillus sp. strain ZI120
<220>
   <221> CDS
   <222> (202)..(834)
<400> 30
   acagatgaag ttgagcaggt tggcgtattc tctattgaag aagatcagca aaaagaagat      60
   tcgactgata ttgatgtaga cattattttt gattacgatt atattcccgt attatcagtt     120
   gagttggacc ctgaagatgt tgatgcatta agtgaagaag atggaatcgc atatattgaa     180
   gaagactttg aagtatcaat c cag caa tcg gtg cct tgg ggt att act cgt       231
                           Gln Gln Ser Val Pro Trp Gly Ile Thr Arg
                           1               5                   10
   gta caa gct cca gca gcg att aac cgt gga aca aat ggt tca gga gta       279
   Val Gln Ala Pro Ala Ala Ile Asn Arg Gly Thr Asn Gly Ser Gly Val
                   15                  20                  25
   aga gtg gct gta ttg gat aca gga att tct aca cat agt gat tta aca       327
   Arg Val Ala Val Leu Asp Thr Gly Ile Ser Thr His Ser Asp Leu Thr
               30                  35                  40
   att cgt ggt gga gct agc ttc gtg cct ggt gaa cca aat aca tct gac       375
   Ile Arg Gly Gly Ala Ser Phe Val Pro Gly Glu Pro Asn Thr Ser Asp
           45                  50                  55
   tta aat ggc cat ggt acc cat gta gct gga aca att gca gct ttg aat      423
   Leu Asn Gly His Gly Thr His Val Ala Gly Thr Ile Ala Ala Leu Asn
       60                  65                  70
   aac tca atc ggc gtt gta ggt gta gca cca aat gct gat cta tat gct      471
   Asn Ser Ile Gly Val Val Gly Val Ala Pro Asn Ala Asp Leu Tyr Ala
   75                  80                  85                  90
   gta aaa gtt ctt ggg gca aat ggt aga gga agc att gga gga att gca      519
   Val Lys Val Leu Gly Ala Asn Gly Arg Gly Ser Ile Gly Gly Ile Ala
                   95                  100                 105
   caa ggt tta gag tgg gca gct gcg aac aat atg cac ata gca aac ttg      567
   Gln Gly Leu Glu Trp Ala Ala Ala Asn Asn Met His Ile Ala Asn Leu
               110                 115                 120
   agc ctt ggt agc gat gca cct agc tca act ctt gag cag gct gtt aat      615
   Ser Leu Gly Ser Asp Ala Pro Ser Ser Thr Leu Glu Gln Ala Val Asn
           125                 130                 135
   tac gct aca agt cgc ggt gta tta gtt att gcg gct tca ggt aat aac      663
   Tyr Ala Thr Ser Arg Gly Val Leu Val Ile Ala Ala Ser Gly Asn Asn
       140                 145                 150
   ggt tca ggt aac gtt gga tat cct gca cgt tat gct aat gca atg gca      711
   Gly Ser Gly Asn Val Gly Tyr Pro Ala Arg Tyr Ala Asn Ala Met Ala
   155                 160                 165                 170
   gta gga gca acc gat caa aat aat aac cgt gct aac ttc tct caa tat      759
   Val Gly Ala Thr Asp Gln Asn Asn Asn Arg Ala Asn Phe Ser Gln Tyr
                   175                 180                 185
   ggt gca gga ctt gat atc gta gct cca ggt gta ggc att caa agt acg      807
   Gly Ala Gly Leu Asp Ile Val Ala Pro Gly Val Gly Ile Gln Ser Thr
               190                 195                 200
   tat cct ggt aac cgc tat gcg agc cta                                  834
   Tyr Pro Gly Asn Arg Tyr Ala Ser Leu
           205                 210
<210> 31
   <211> 211
   <212> PRT
   <213> Bacillus sp. strain ZI120
<400> 31
   Gln Gln Ser Val Pro Trp Gly Ile Thr Arg Val Gln Ala Pro Ala Ala
   1               5                   10                  15
   Ile Asn Arg Gly Thr Asn Gly Ser Gly Val Arg Val Ala Val Leu Asp
               20                  25                  30
   Thr Gly Ile Ser Thr His Ser Asp Leu Thr Ile Arg Gly Gly Ala Ser
   35 40 45
   Phe Val Pro Gly Glu Pro Asn Thr Ser Asp Leu Asn Gly His Gly Thr
   50 55 60
   His Val Ala Gly Thr Ile Ala Ala Leu Asn Asn Ser Ile Gly Val Val
   65 70 75 80
   Gly Val Ala Pro Asn Ala Asp Leu Tyr Ala Val Lys Val Leu Gly Ala
   85 90 95
   Asn Gly Arg Gly Ser Ile Gly Gly Ile Ala Gln Gly Leu Glu Trp Ala
   100 105 110
   Ala Ala Asn Asn Met His Ile Ala Asn Leu Ser Leu Gly Ser Asp Ala
   115 120 125
   Pro Ser Ser Thr Leu Glu Gln Ala Val Asn Tyr Ala Thr Ser Arg Gly
   130 135 140
   Val Leu Val Ile Ala Ala Ser Gly Asn Asn Gly Ser Gly Asn Val Gly
   145 150 155 160
   Tyr Pro Ala Arg Tyr Ala Asn Ala Met Ala Val Gly Ala Thr Asp Gln
   165 170 175
   Asn Asn Asn Arg Ala Asn Phe Ser Gln Tyr Gly Ala Gly Leu Asp Ile
   180 185 190
   Val Ala Pro Gly Val Gly Ile Gln Ser Thr Tyr Pro Gly Asn Arg Tyr
   195 200 205
   Ala Ser Leu
   210
<210> 32
   <211> 837
   <212> DNA
   <213> Bacillus sp. strain ZI130
<220>
   <221> CDS
   <222> (205) .. (837)
<400> 32
   tttacagatg aagttgagca ggttggcgta ttctctattg aagaagatca gcaaaaagaa 60
   gattcgactg atattgatgt agacattatt tttgattacg attatattcc cgtattatca 120
   gttgagttgg accctgaaga tgttgatgca ttaagtgaag aagatggaat cgcatatatt 180
   gaagaagact ttgaggtatc aatc cag caa tcg gtg cct tgg ggt att act 231
   Gln Gln Ser Val Pro Trp Gly Ile Thr
   1 5
   cgt gta caa gct cca gca gcg att aac cgt gga aca aat ggt tca gga 279
   Arg Val Gln Ala Pro Ala Ala Ile Asn Arg Gly Thr Asn Gly Ser Gly
   10 15 20 25
   gta aga gtg gct gta ttg gat aca gga att tct aca cat agt gat tta 327
   Val Arg Val Ala Val Leu Asp Thr Gly Ile Ser Thr His Ser Asp Leu
   30 35 40
   aca att cgt ggt gga gct agc ttc gtg cct ggt gaa cca aat aca tct 375
   Thr Ile Arg Gly Gly Ala Ser Phe Val Pro Gly Glu Pro Asn Thr Ser
   45 50 55
   gac tta aat ggc cat ggt acc cat gta gct gga aca att gca gct ttg 423
   Asp Leu Asn Gly His Gly Thr His Val Ala Gly Thr Ile Ala Ala Leu
   60 65 70
   aat aac tca atc ggc gtt gta ggt gta gca cca aat gct gat cta tat 471
   Asn Asn Ser Ile Gly Val Val Gly Val Ala Pro Asn Ala Asp Leu Tyr
   75 80 85
   gct gta aaa gtt ctt ggg gca aat ggt aga gga agc att gga gga att 519
   Ala Val Lys Val Leu Gly Ala Asn Gly Arg Gly Ser Ile Gly Gly Ile
   90 95 100 105
   gca caa ggt tta gag tgg gca gct gcg aac aat atg cac ata gca aac 567
   Ala Gln Gly Leu Glu Trp Ala Ala Ala Asn Asn Met His Ile Ala Asn
   110 115 120
   ttg agc ctt ggt agc gat gca cct agc tca act ctt gag cag gct gtt 615
   Leu Ser Leu Gly Ser Asp Ala Pro Ser Ser Thr Leu Glu Gln Ala Val
   125 130 135
   aat tac gct aca agt cgc ggt gta tta gtt att gcg gct tca ggt aat 663
   Asn Tyr Ala Thr Ser Arg Gly Val Leu Val Ile Ala Ala Ser Gly Asn
   140 145 150
   aac ggt tca ggt aac gtt gga tat cct gca cgt tat gct aat gca atg 711
   Asn Gly Ser Gly Asn Val Gly Tyr Pro Ala Arg Tyr Ala Asn Ala Met
   155 160 165
   gca gta gga gca acc gat caa aat aat aac cgt gct aac ttc tct caa 759
   Ala Val Gly Ala Thr Asp Gln Asn Asn Asn Arg Ala Asn Phe Ser Gln
   170 175 180 185
   tat ggt gca gga ctt gat atc gta gct cca ggt gta ggc att caa agt 807
   Tyr Gly Ala Gly Leu Asp Ile Val Ala Pro Gly Val Gly Ile Gln Ser
   190 195 200
   acg tat cct ggt aac cgc tat gcg agc cta 837
   Thr Tyr Pro Gly Asn Arg Tyr Ala Ser Leu
   205 210
<210> 33
   <211> 211
   <212> PRT
   <213> Bacillus sp. strain ZI130
<400> 33
   Gln Gln Ser Val Pro Trp Gly Ile Thr Arg Val Gln Ala Pro Ala Ala
   1 5 10 15
   Ile Asn Arg Gly Thr Asn Gly Ser Gly Val Arg Val Ala Val Leu Asp
   20 25 30
   Thr Gly Ile Ser Thr His Ser Asp Leu Thr Ile Arg Gly Gly Ala Ser
   35 40 45
   Phe Val Pro Gly Glu Pro Asn Thr Ser Asp Leu Asn Gly His Gly Thr
   50 55 60
   His Val Ala Gly Thr Ile Ala Ala Leu Asn Asn Ser Ile Gly Val Val
   65 70 75 80
   Gly Val Ala Pro Asn Ala Asp Leu Tyr Ala Val Lys Val Leu Gly Ala
   85 90 95
   Asn Gly Arg Gly Ser Ile Gly Gly Ile Ala Gln Gly Leu Glu Trp Ala
   100 105 110
   Ala Ala Asn Asn Met His Ile Ala Asn Leu Ser Leu Gly Ser Asp Ala
   115 120 125
   Pro Ser Ser Thr Leu Glu Gln Ala Val Asn Tyr Ala Thr Ser Arg Gly
   130 135 140
   Val Leu Val Ile Ala Ala Ser Gly Asn Asn Gly Ser Gly Asn Val Gly
   145 150 155 160
   Tyr Pro Ala Arg Tyr Ala Asn Ala Met Ala Val Gly Ala Thr Asp Gln
   165 170 175
   Asn Asn Asn Arg Ala Asn Phe Ser Gln Tyr Gly Ala Gly Leu Asp Ile
   180 185 190
   Val Ala Pro Gly Val Gly Ile Gln Ser Thr Tyr Pro Gly Asn Arg Tyr
           195                 200                 205
   Ala Ser Leu
       210
<210> 34
   <211> 837
   <212> DNA
   <213> Bacillus sp. strain ZI132
<220>
   <221> CDS
   <222> (202) .. (837)
<400> 34
   acagatgaag ttgaacaggt tggcgtattc tctattgaag aagatcagca aaaagaagat       60
   tcgactgata ttgatgtaga cattattttt gattacgatt atattcccgt attatcagtt      120
   gaattggatc ctgaagatgt tgatgcatta agtgaagaag atggaatcgc atatattgaa      180
   gaagactttg aggtatcaat t cag caa tcg gtg cct tgg ggt att aat cgt        231
                           Gln Gln Ser Val Pro Trp Gly Ile Asn Arg
                           1               5                   10
   gta caa gct cca aca gcg att aac cgt gga aca aat ggt tca gga gta        279
   Val Gln Ala Pro Thr Ala Ile Asn Arg Gly Thr Asn Gly Ser Gly Val
                   15                  20                  25
   aga gtg gct gta ttg gat aca gga att tct aca cat agt gat tta aca        327
   Arg Val Ala Val Leu Asp Thr Gly Ile Ser Thr His Ser Asp Leu Thr
               30                  35                  40
   att cgt ggt gga gct agc ttc gtg cct ggt gaa cca aat aca tct gac        375
   Ile Arg Gly Gly Ala Ser Phe Val Pro Gly Glu Pro Asn Thr Ser Asp
           45                  50                  55
   tta aat ggc cat ggt act cat gta gcg gga aca att gca gct ttg aat        423
   Leu Asn Gly His Gly Thr His Val Ala Gly Thr Ile Ala Ala Leu Asn
       60                  65                  70
   aac tca att ggc gtt gta gga gta gca cca aat gct gat cta tat gct        471
   Asn Ser Ile Gly Val Val Gly Val Ala Pro Asn Ala Asp Leu Tyr Ala
   75                  80                  85                  90
   gta aaa gtt ctt ggg gca aat ggt aga gga agc att ggc gga att gca        519
   Val Lys Val Leu Gly Ala Asn Gly Arg Gly Ser Ile Gly Gly Ile Ala
                   95                  100                 105
   caa ggt tta gag tgg gca gct gct aac aat atg cac ata gca aac ttg        567
   Gln Gly Leu Glu Trp Ala Ala Ala Asn Asn Met His Ile Ala Asn Leu
               110                 115                 120
   agc ctt ggt agc gat gca cct agc tca act ctt gag cag gct gtt aat        615
   Ser Leu Gly Ser Asp Ala Pro Ser Ser Thr Leu Glu Gln Ala Val Asn
           125                 130                 135
   tac gct aca agt cgc ggt gta tta gtt att gcg gct tca ggt aat aac      663
   Tyr Ala Thr Ser Arg Gly Val Leu Val Ile Ala Ala Ser Gly Asn Asn
       140                 145                 150
   ggt tca ggt aac gtt gga tat cct gca cgt tat gct aat gca atg gca      711
   Gly Ser Gly Asn Val Gly Tyr Pro Ala Arg Tyr Ala Asn Ala Met Ala
   155                 160                 165                 170
   gta gga gca acc gat caa aat aat aac cgt gct aac ttc tct caa tac      759
   Val Gly Ala Thr Asp Gln Asn Asn Asn Arg Ala Asn Phe Ser Gln Tyr
                   175                 180                 185
   ggt gca gga ctt gat atc gta gct cca ggt gta ggc att caa agt acg      807
   Gly Ala Gly Leu Asp Ile Val Ala Pro Gly Val Gly Ile Gln Ser Thr
               190                 195                 200
   tac cct ggt aac cgc tat gcg agt cta atg                              837
   Tyr Pro Gly Asn Arg Tyr Ala Ser Leu Met
           205                 210
<210> 35
   <211> 212
   <212> PRT
   <213> Bacillus sp. strain ZI132
<400> 35
   Gln Gln Ser Val Pro Trp Gly Ile Asn Arg Val Gln Ala Pro Thr Ala
   1               5                   10                  15
   Ile Asn Arg Gly Thr Asn Gly Ser Gly Val Arg Val Ala Val Leu Asp
               20                  25                  30
   Thr Gly Ile Ser Thr His Ser Asp Leu Thr Ile Arg Gly Gly Ala Ser
           35                  40                  45
   Phe Val Pro Gly Glu Pro Asn Thr Ser Asp Leu Asn Gly His Gly Thr
       50                  55                  60
   His Val Ala Gly Thr Ile Ala Ala Leu Asn Asn Ser Ile Gly Val Val
   65                  70                  75                  80
   Gly Val Ala Pro Asn Ala Asp Leu Tyr Ala Val Lys Val Leu Gly Ala
                   85                  90                  95
   Asn Gly Arg Gly Ser Ile Gly Gly Ile Ala Gln Gly Leu Glu Trp Ala
               100                 105                 110
   Ala Ala Asn Asn Met His Ile Ala Asn Leu Ser Leu Gly Ser Asp Ala
           115                 120                 125
   Pro Ser Ser Thr Leu Glu Gln Ala Val Asn Tyr Ala Thr Ser Arg Gly
       130                 135                 140
   Val Leu Val Ile Ala Ala Ser Gly Asn Asn Gly Ser Gly Asn Val Gly
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Asn Phe Ser Gln Tyr Gly Ala Gly Leu Asp Ile
               180                 185                 190
   Val Ala Pro Gly Val Gly Ile Gln Ser Thr Tyr Pro Gly Asn Arg Tyr
           195                 200                 205
   Ala Ser Leu Met
       210
<210> 36
   <211> 801
   <212> DNA
   <213> Bacillus sp. strain ZI340
<220>
   <221> CDS
   <222> (154) .. (801)
<400> 36
   actcagcatg atgatgaggc tattgatgtt gatattattt atgattatga ttatatccca      60
   gtcttatcag tagagatcga tcctgaagat gtcgaggtac tcagtcaaga agaaggcatt     120
   gcctatattg aggaagactt tgaagtatcc att caa cag act gta cct tgg ggc      174
                                        Gln Gln Thr Val Pro Trp Gly
                                        1               5
   att caa aga gta caa gct cct gca gtt att aat cgt ggc att aat ggc      222
   Ile Gln Arg Val Gln Ala Pro Ala Val Ile Asn Arg Gly Ile Asn Gly
           10                  15                  20
   agt ggg gta cga gta gcg gtg ctt gat tca ggc att tcc act cat agt      270
   Ser Gly Val Arg Val Ala Val Leu Asp Ser Gly Ile Ser Thr His Ser
       25                  30                  35
   gat tta agc att tcc ggt ggc gta agc ttt gtc cct ggt gaa cca act      318
   Asp Leu Ser Ile Ser Gly Gly Val Ser Phe Val Pro Gly Glu Pro Thr
   40                  45                  50                  55
   att tct gat gga aat ggc cat ggt aca cat gta gcg gga acg att gct      366
   Ile Ser Asp Gly Asn Gly His Gly Thr His Val Ala Gly Thr Ile Ala
                   60                  65                  70
   gca ctt aat aac agc att ggt gtg gta ggt gtt gca ccg aat gct caa      414
   Ala Leu Asn Asn Ser Ile Gly Val Val Gly Val Ala Pro Asn Ala Gln
               75                  80                  85
   att tat gga gta aaa gtt cta gga gca aac ggt cgc gga agt gtg agc      462
   Ile Tyr Gly Val Lys Val Leu Gly Ala Asn Gly Arg Gly Ser Val Ser
           90                  95                  100
   ggt att gct cag gga tta gag tgg gcc gct aca aac aat atg gat att      510
   Gly Ile Ala Gln Gly Leu Glu Trp Ala Ala Thr Asn Asn Met Asp Ile
       105                 110                 115
   gca aac tta agc cta gga agt gac gca cca agc tca act ctt gaa caa      558
   Ala Asn Leu Ser Leu Gly Ser Asp Ala Pro Ser Ser Thr Leu Glu Gln
   120                 125                 130                 135
   gct gtt aac ttt gcc acg agc aga ggt gta ctt gtt gtt gca gct tca      606
   Ala Val Asn Phe Ala Thr Ser Arg Gly Val Leu Val Val Ala Ala Ser
                   140                 145                 150
   gga aat aac ggg tct gga aac gtt ggc ttc cct gca cgt tac gca aat      654
   Gly Asn Asn Gly Ser Gly Asn Val Gly Phe Pro Ala Arg Tyr Ala Asn
               155                 160                 165
   gca atg gca gtt gga gca aca gat caa aac aat aga cgc gct aac ttt      702
   Ala Met Ala Val Gly Ala Thr Asp Gln Asn Asn Arg Arg Ala Asn Phe
           170                 175                 180
   tca caa tat gga gca ggt ctt gat att gta gct cct gga gta ggt gta      750
   Ser Gln Tyr Gly Ala Gly Leu Asp Ile Val Ala Pro Gly Val Gly Val
       185                 190                 195
   caa agt aca tat cca ggc aat cgt tat gta agt atg aat agt aca tct      798
   Gln Ser Thr Tyr Pro Gly Asn Arg Tyr Val Ser Met Asn Ser Thr Ser
   200                 205                 210                 215
   aag                                                                  801
   Lys
<210> 37
   <211> 216
   <212> PRT
   <213> Bacillus sp. strain ZI340
<400> 37
   Gln Gln Thr Val Pro Trp Gly Ile Gln Arg Val Gln Ala Pro Ala Val
   1               5                   10                  15
   Ile Asn Arg Gly Ile Asn Gly Ser Gly Val Arg Val Ala Val Leu Asp
               20                  25                  30
   Ser Gly Ile Ser Thr His Ser Asp Leu Ser Ile Ser Gly Gly Val Ser
           35                  40                  45
   Phe Val Pro Gly Glu Pro Thr Ile Ser Asp Gly Asn Gly His Gly Thr
       50                  55                  60
   His Val Ala Gly Thr Ile Ala Ala Leu Asn Asn Ser Ile Gly Val Val
   65                  70                  75                  80
   Gly Val Ala Pro Asn Ala Gln Ile Tyr Gly Val Lys Val Leu Gly Ala
                   85                  90                  95
   Asn Gly Arg Gly Ser Val Ser Gly Ile Ala Gln Gly Leu Glu Trp Ala
               100                 105                 110
   Ala Thr Asn Asn Met Asp Ile Ala Asn Leu Ser Leu Gly Ser Asp Ala
           115                 120                 125
   Pro Ser Ser Thr Leu Glu Gln Ala Val Asn Phe Ala Thr Ser Arg Gly
       130                 135                 140
   Val Leu Val Val Ala Ala Ser Gly Asn Asn Gly Ser Gly Asn Val Gly
   145                 150                 155                 160
   Phe Pro Ala Arg Tyr Ala Asn Ala Met Ala Val Gly Ala Thr Asp Gln
                   165                 170                 175
   Asn Asn Arg Arg Ala Asn Phe Ser Gln Tyr Gly Ala Gly Leu Asp Ile
               180                 185                 190
   Val Ala Pro Gly Val Gly Val Gln Ser Thr Tyr Pro Gly Asn Arg Tyr
           195                 200                 205
   Val Ser Met Asn Ser Thr Ser Lys
       210                 215

## Description

### SEQUENCES

This application contains the following sequences:
SEQ ID NO:1 - DNA encoding subtilase from Bacillus sp. strain Zi344. Nucleic acids 337 to 1143 encodes the mature subtilase.
SEQ ID NO:2 - Amino acid sequence of subtilase from Bacillus sp. strain Zi344. The mature subtilase is amino acids 113 to 381.
SEQ ID NO:3 - DNA encoding subtilase from Bacillus sp. strain EP655. Nucleic acids 343 to 1149 encodes the mature subtilase.
SEQ ID NO:4 - Amino acid sequence of subtilase from Bacillus sp. strain EP655. The mature subtilase is amino acids 115 to 383.
SEQ ID NO:5 - DNA encoding subtilase from Bacillus sp. strain p203. Nucleic acids 343 to 1149 encodes the mature subtilase.
SEQ ID NO:6 - Amino acid sequence of subtilase from Bacillus sp. strain p203. The mature subtilase is amino acids 115 to 383.
SEQ ID NO:7 to SEQ ID NO:27 are artificial primers.
SEQ ID NO:28 - Partial DNA sequence encoding subtilase from Bacillus sp. strain EP63.
SEQ ID NO:29 - Partial amino acid sequence of subtilase from Bacillus sp. strain EP63.
SEQ ID NO:30 - Partial DNA sequence encoding subtilase from Bacillus sp. strain ZI120.
SEQ ID NO:31 - Partial amino acid sequence of subtilase from Bacillus sp. strain ZI120.
SEQ ID NO:32 - Partial DNA sequence encoding subtilase from Bacillus sp. strain ZI130.
SEQ ID NO:33 - Partial amino acid sequence of subtilase from Bacillus sp. strain ZI130.
SEQ ID NO:34 - Partial DNA sequence encoding subtilase from Bacillus sp. strain ZI132.
SEQ ID NO:35 - Partial amino acid sequence of subtilase from Bacillus sp. strain ZI132.
SEQ ID NO:36 - Partial DNA sequence encoding subtilase from Bacillus sp. strain ZI340.
SEQ ID NO:37 - Partial amino acid sequence of subtilase from Bacillus sp. strain ZI340.
The amino acid sequences of SEQ ID NO:29, 31, 33, 35 and 37 are mature subtilases where the C-terminals are truncated.

### DEPOSITED MICROORGANISMS

The wild type strain referred to as p203 was deposited on 23 June 2005 under the Budapest treaty at the Deutsche Sammlung von Mikroorganismen und Zellkulturen under the deposit number DSM 17419. The deposit contains the subtilase gene referred to as p203A herein, which is identical with SEQ ID NO:5.

### FIELD OF THE INVENTION

The present invention relates to novel subtilases from wild-type strains of Bacillus and to methods of construction and production of these proteases. Further, the present invention relates to use of the claimed subtilases in detergents, such as a laundry detergent or an automatic dishwashing detergent.

### BACKGROUND OF THE INVENTION

Enzymes have been used within the detergent industry as part of washing formulations for more than 30 years. Proteases are from a commercial perspective the most relevant enzyme in such formulations, but other enzymes including lipases, amylases, cellulases, hemicellulases or mixtures of enzymes are also often used.

The search for proteases with appropriate properties include both discovery of naturally occurring proteases, i.e. so called wild-type proteases but also alteration of well-known proteases by e.g. genetic manipulation of the nucleic acid sequence encoding said proteases. One family of proteases, which is often used in detergents, is the subtilases. This family has been further grouped into 6 different sub-groups (Siezen R.J. and Leunissen J.A.M., 1997, Protein Science, 6, 501-523). One of these sub-groups, the Subtilisin family was further divided into the subgroups of "true subtilisins (I-S1)", "high alkaline proteases (IS2)" and "intracellular proteases". Siezen and Leunissen identified also some proteases of the subtilisin family, but not belonging to any of the subgroups. The true subtilisins include proteases such as subtilisin BPN' (BASBPN), subtilisin Carlsberg (ALCALASE^{®}, NOVOZYMES A/S) (BLSCAR), mesentericopeptidase (BMSAMP) and subtilisin DY (BSSDY). The high alkaline proteases include proteases such as subtilisin 309 (SAVINASE^{®}, NOVOZYMES A/S) (BLSAVI) subtilisin PB92 (BAALKP), subtilisin BL or BLAP (BLSUBL), subtilisin 147 (ESPERASE^{®}, NOVOZYMES A/S), subtilisin Sendai (BSAPRS) and alkaline elastase YaB. Outside this grouping of the subtilisin family a further subtilisin subgroup was recently identified on the basis of the 3-D structure of its members, the TY145 like subtilisins. The TY145 like subtilisins include proteases such as TY145 (a subtilase from Bacillus sp. TY145, NCIMB 40339 described in WO 92/17577) (BSTY145), subtilisin TA41 (BSTA41), and subtilisin TA39 (BSTA39).

The PD138 type of protease was first described physico-chemically in WO 93/18140 to Novo Nordisk A/S disclosing one strain producing this type of protease. In WO 93/18140, PD138 type of protease was described based on immunological cross reaction with a polyclonal rabbit antibody directed towards the purified protease. The primary structure of the protease was not disclosed. Later the Bacillus species producing this protease was taxonomically classified as *Bacillus gibsonii* (Nielsen et al. 1995). The type strain of *Bacillus gibsonii* is identical with the strain described in WO 93/18140. WO 2003/054184 and WO 2003/054185 disclose alkaline subtilases from strains of *Bacillus gibsonii.*

### BRIEF DESCRIPTION OF THE INVENTION

The inventors have isolated novel proteases belonging to the PD138 like proteases subgroup of the subtilisin family that possess advantageous properties, such as improved performance in detergent at low temperature.

### BRIEF DESCRIPTION OF DRAWINGS

Figure 1: Phylogenetic tree showing the relationship of the mature subtilase peptide sequences were constructed upon alignment with default settings in the ClustaIV function of program MegAlign^{™} version 5.05 in DNAStar^{™} program package.
Figure 2: The alignment of the sequences from the PCR screening from figure 1.

### DEFINITIONS

Prior to discussing this invention in further detail, the following terms and conventions will first be defined.

The term "subtilases" refer to a sub-group of serine proteases according to Siezen et al., Protein Engng. 4 (1991) 719-737 and Siezen et al. Protein Science 6 (1997) 501-523. Serine proteases or serine peptidases is a subgroup of proteases characterised by having a serine in the active site, which forms a covalent adduct with the substrate. Further the subtilases (and the serine proteases) are characterised by having two active site amino acid residues apart from the serine, namely a histidine and an aspartic acid residue.
The subtilases may be divided into 6 sub-divisions, i.e. the Subtilisin family, the Thermitase family, the Proteinase K family, the Lantibiotic peptidase family, the Kexin family and the Pyrolysin family.

The Subtilisin family (EC 3.4.21.62) may be further divided into 3 sub-groups, i.e. I-S1 ("true" subtilisins), I-S2 (highly alkaline proteases) and intracellular subtilisins. Definitions or grouping of enzymes may vary or change, however, in the context of the present invention the above division of subtilases into sub-division or sub-groups shall be understood as those described by Siezen et al., Protein Engng. 4 (1991) 719-737 and Siezen et al. Protein Science 6 (1997) 501-523.

The term "parent" is in the context of the present invention to be understood as a protein, which is modified to create a protein variant. The parent protein may be a naturally occurring (wild-type) polypeptide or it may be a variant thereof prepared by any suitable means. For instance, the parent protein may be a variant of a naturally occurring protein which has been modified by substitution, chemical modification, deletion or truncation of one or more amino acid residues, or by addition or insertion of one or more amino acid residues to the amino acid sequence, of a naturally-occurring polypeptide. Thus the term "parent subtilase" refers to a subtilase which is modified to create a subtilase variant.

"Homology" or "homologous to" is in the context of the present invention to be understood in its conventional meaning and the "homology" between two amino acid sequences should be determined by use of the "Similarity" defined by the GAP program from the University of Wisconsin Genetics Computer Group (UWGCG) package using default settings for alignment parameters, comparison matrix, gap and gap extension penalties. Default values for GAP penalties, i.e. GAP creation penalty of 3.0 and GAP extension penalty of 0.1 (Program Manual for the Wisconsin Package, Version 8, August 1994, Genetics Computer Group, 575 Science Drive, Madison, Wisconsin, USA 53711). The method is also described in S.B. Needleman and C.D. Wunsch, Journal of Molecular Biology, 48, 443-445 (1970). Identities can be extracted from the same calculation. The homology between two amino acid sequences can also be determined by "identity" or "similarity" using the GAP routine of the UWGCG package version 9.1 with default setting for alignment parameters, comparison matrix, gap and gap extension penalties can also be applied using the following parameters: gap creation penalty = 8 and gap extension penalty = 8 and all other parameters kept at their default values. The output from the routine is besides the amino acid alignment the calculation of the "Percent Identity" and the "Similarity" between the two sequences. The numbers calculated using UWGCG package version 9.1 is slightly different from the version 8.

The term "position" is in the context of the present invention to be understood as the number of an amino acid in a peptide or polypeptide when counting from the N-terminal end of said peptide/polypeptide. The position numbers used in the present invention refer to different subtilases depending on which subgroup the subtilase belongs to.

### DETAILED DESCRIPTION OF THE INVENTION

### Selection of strains producing novel subtilisins

In the search for Bacillus strains producing novel subtilases we selected a number of strains, which based on 16S rDNA similarity was related to *Bacillus gibsonii.* The Bacillus strains P203, EP655, ZI344, EP63, ZI120, ZI130, ZI132 and ZI140 were fermented in a standard Bacillus fermentation medium (BP-X added 0.1 M NaHCO3 to adjust pH to 9).

The immunochemical properties can be determined immunologically by cross-reaction identity tests. The identity test can be performed either by the well known ouchterlony double immuno diffusion procedure or by tandem crossed immunoelectro-phoresis according to N.H Axelsen, Handbook of immunoprecipitation-in-gel Techniques. Blackwell Scientific Publications (1983) chapters 5 &14. The terms "antigenic identity" and "partial antigenic identity" are described in the same book chapters 5, 19 and 20.

Culture fluids were analysed for protease activity using Alcalase^{™} as standard. Fluids with 10 CPU/L or more activity was included in the immunological analysis. The analysis included two different polyclonal rabbit antibodies; AB41 was antibody raised against the PD138 protease (WO 93/18140). The other antibody was AB65 raised against PD490 protease (Not published). The analysis gave two groups of proteases with a partial reaction against the AB41. One of these groups also has a partial reaction against AB65, whereas the other group reacted identical with AB65. A third group including PD138 gave identical reaction with AB41 and partial reaction with AB65.

### PCR screening

A part of the genes encoding the proteases which exhibited novel immunochemical properties as described above was amplified with a standard PCR reaction with PCR primers designed from available sequences, see Example 1.

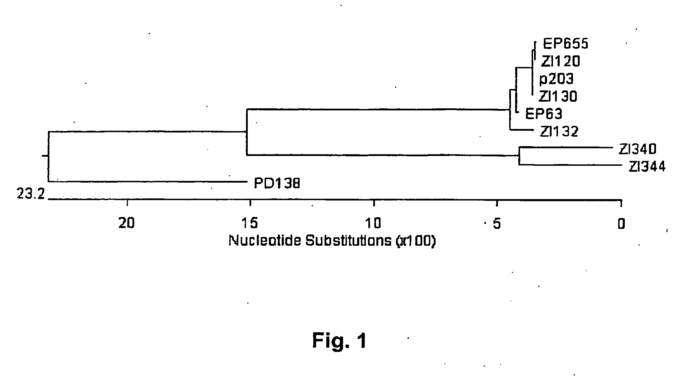

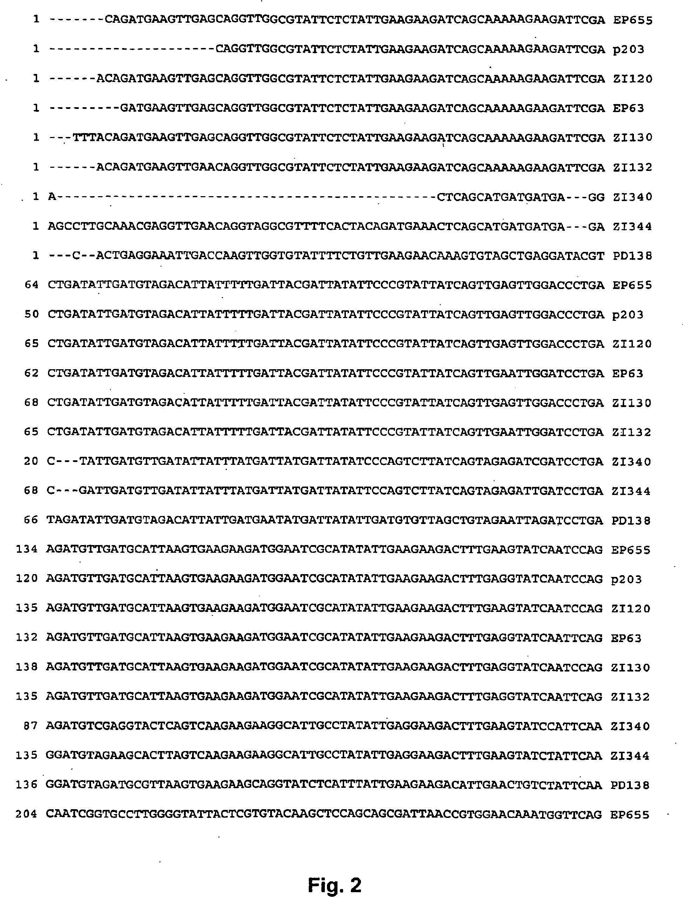

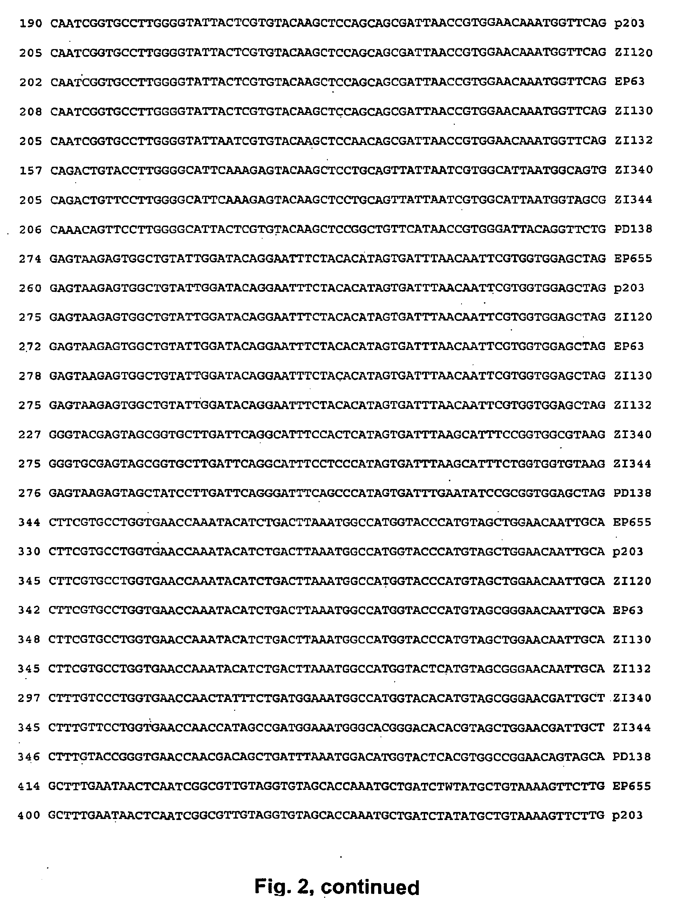

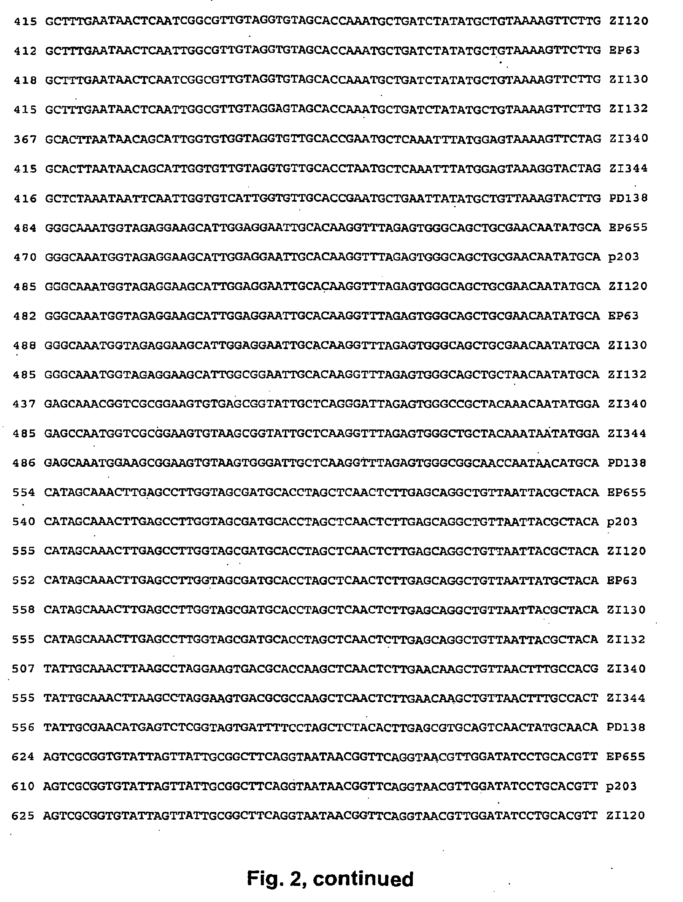

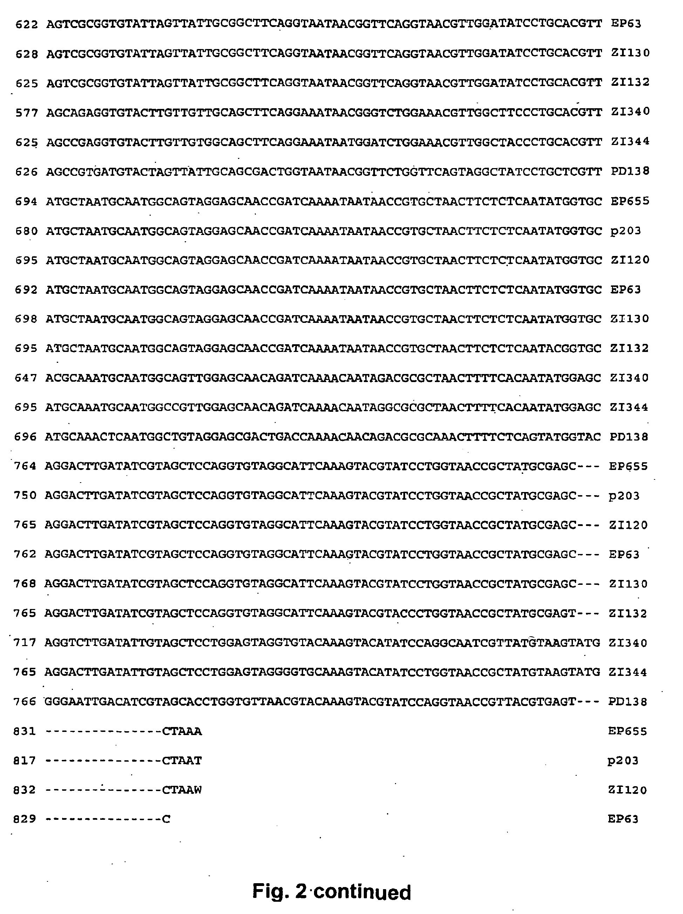

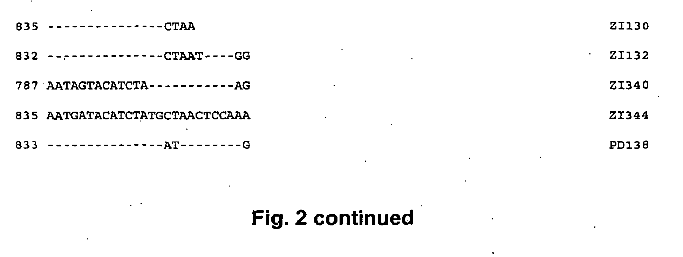

The nucleotide sequences were analysed with DNA STAR^{™}, and based on nucleotide sequence diversity with PD138 as benchmark the novel groups identified with antibodies were confirmed. A phylogenetic tree based on the sequences from the PCR screening is presented in Figure 1. A ClustaIV alignment of the sequences from the PCR screening is shown in Figure 2.

### Cloning and expression of full length subtilase of the invention

### Inverse PCR

Inverse PCR was performed with specific DNA primers designed to complement the DNA sequence obtained from PCR product of the partial protease gene and chromosomal DNA extracted from the appropriate bacterial strain. Inverse PCR was made on the strains P203, EP655 and ZI344, whereas the strains EP63, ZI120, ZI130, ZI1342 and ZI140 were not further investigated. The inverse PCR products were nucleotide sequenced to obtain the region encoding the N and C terminal parts of the genes.

### Production of full length subtilase

The subtilase genes were amplified with specific primers with restriction sites in the 5' end of primers that allow gene fusion with the Savinase signal peptide of plasmid pDG268NeoMCS-PramyQ/PrcryIII/cryIIIAstab/Sav (US 5,955,310). Protease positive colonies were selected and the coding sequence of the expressed enzyme from the expression construct was confirmed by DNA sequence analysis.

### Subtilases of the invention

The subtilases of the present invention include subtilases from the Bacillus strain ZI344 as shown in SEQ ID NO:2. WO 2003/054184 discloses an alkaline protease from *Bacillus gibsonii,* DSM 14393 which has app. 85.9% amino acid sequence identity with ZI344 and app. 87% amino acid sequence identity with EP655 and P203. Further, the alkaline protease from *Bacillus gibsonii,* DSM 14393 has 88.2% identity with the partial sequence of the subtilases from ZI120 and ZI130 (SEQ ID NO:31 and 33); and 88.1%, 86.8% and 83.8% identity with the partial sequence of the subtilases from EP63, ZI132 and ZI340 (SEQ ID NO:29, 35 and 37) respectively.

The protease from *Bacillus gibsonii,* DSM 14393 is encoded by a nucleic acid sequence which is app. 75.5% identical with SEQ ID NO;1 and app. 80.2% identical with SEQ ID NO's: 3 and 5. The nucleic acid sequence encoding the protease from *Bacillus gibsonii,* DSM 14393 is 72.2%, 75.7%, 75.7%, 76.2% and 75.5% identical with the nucleic acid sequence encoding the mature part of the partial sequence of the subtilases from ZI340 (SEQ ID NO:36), ZI120 (SEQ ID NO:30), ZI130 (SEQ ID NO:32), EP63 (SEQ ID NO:28) and ZI132 (SEQ ID NO:34) respectively.

Thus, the subtilase of the present invention is at least 90% identical with SEQ ID NO:2. Preferably, said subtilase is at least 91% identical with SEQ ID NO:2, more preferably said subtilase is at least 92%, 93%, 94%, 95%, 96%, 97%, 98% or at least 99% identical with SEQ ID NO:2.

Correspondingly, the subtilases according to the present invention are encoded by an isolated nucleic acid sequence as shown in SEQ ID NO:1. Preferably, said nucleic acid sequence is at least 81% identical with SEQ ID NO:1, more preferably said nucleic acid sequence is at least 82%, 83%, 84%, 85%, 86%, 87%, 88%, 89%, 90%, 91%, 92%, 93%, 94%, 95%, 96%, 97%, 98% or at least 99% identical with SEQ ID NO:1.

Further the isolated nucleic acid sequence encoding a subtilase of the invention hybridizes with a complementary strand of the nucleic acid sequence shown in SEQ ID NO:1 under low stringency conditions, at least under medium stringency conditions, at least under medium/high stringency conditions, at least under high stringency conditions, at least under very high stringency conditions as described below.

### Hybridization

Suitable experimental conditions for determining hybridization between a nucleotide probe and a homologous DNA or RNA sequence involves presoaking of the filter containing the DNA fragments or RNA to hybridize in 5 x SSC (Sodium chloride/Sodium citrate, Sambrook et al. 1989) for 10 min, and prehybridization of the filter in a solution of 5 x SSC, 5 x Denhardt's solution (Sambrook et al. 1989), 0.5 % SDS and 100 µg/ml of denatured sonicated salmon sperm DNA (Sambrook et al. 1989), followed by hybridization in the same solution containing a concentration of 10ng/ml of a random-primed (Feinberg, A. P. and Vogelstein, B. (1983) Anal. Biochem. 132:6-13), ³²P-dCTP-labeled (specific activity > 1 x 10⁹ cpm/µg) probe for 12 hours at ca. 45°C. For various stringency conditions the filter is then washed twice for 30 minutes in 2 x SSC, 0.5 % SDS and at least 55°C (low stringency), more preferably at least 60°C (medium stringency), still more preferably at least 65°C (medium/high stringency), even more preferably at least 70°C (high stringency), and even more preferably at least 75°C (very high stringency).

### VARIANTS

### Combined modifications

The present invention also encompasses any of the above mentioned subtilase variants in combination with any other modification to the amino acid sequence thereof. Especially combinations with other modifications known in the art to provide improved properties to the enzyme are envisaged.

Such combinations comprise the positions: 222 (improves oxidation stability), 218 (improves thermal stability), substitutions in the Ca²⁺-binding sites stabilizing the enzyme, e.g. position 76, and many other apparent from the prior art. In further embodiments a subtilase variant described herein may advantageously be combined with one or more modification(s) in any of the positions: 27, 36, 56, 76, 87, 95, 96, 97, 98, 99, 100, 101, 102, 103, 104, 120, 123, 167, 170, 206, 218, 222, 224, 232, 235, 236, 245, 248, 252 and 274 (BPN' numbering). The novel subtilases differ from the primary structure of BPN' by deletion at the following positions 36, 57 and 158 to 162. The novel subtilase are 6 amino acids shorter than BPN'.

### Methods for expression and isolation of proteins

To express an enzyme of the present invention the above mentioned host cells transformed or transfected with a vector comprising a nucleic acid sequence encoding an enzyme of the present invention are typically cultured in a suitable nutrient medium under conditions permitting the production of the desired molecules, after which these are recovered from the cells, or the culture broth.

The medium used to culture the host cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The media may be prepared using procedures known in the art (see, e.g., references for bacteria and yeast; Bennett, J.W. and LaSure, L., editors, More Gene Manipulations in Fungi, Academic Press, CA, 1991).

If the enzymes of the present invention are secreted into the nutrient medium, they may be recovered directly from the medium. If they are not secreted, they may be recovered from cell lysates. The enzymes of the present invention may be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. ion exchange chromatography, gelfiltration chromatography, affinity chromatography, or the like, dependent on the enzyme in question.

The enzymes of the invention may be detected using methods known in the art that are specific for these proteins. These detection methods include use of specific antibodies, formation of a product, or disappearance of a substrate. For example, an enzyme assay may be used to determine the activity of the molecule. Procedures for determining various kinds of activity are known in the art.

The enzymes of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing (IEF), differential solubility (e.g., ammonium sulfate precipitation), or extraction (see, e.g., Protein Purification, J-C Janson and Lars Ryden, editors, VCH Publishers, New York, 1989).

When an expression vector comprising a DNA sequence encoding an enzyme of the present invention is transformed/transfected into a heterologous host cell it is possible to enable heterologous recombinant production of the enzyme. An advantage of using a heterologous host cell is that it is possible to make a highly purified enzyme composition, characterized in being free from homologous impurities, which are often present when a protein or peptide is expressed in a homologous host cell. In this context homologous impurities mean any impurity (e.g. other polypeptides than the enzyme of the invention) which originates from the homologous cell where the enzyme of the invention is originally obtained from.

### DETERGENT APPLICATIONS

The enzyme of the invention may be added to and thus become a component of a detergent composition.

The detergent composition of the invention may for example be formulated as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations, especially for automatic dish washing (ADW).

In a specific aspect, the invention provides a detergent additive comprising the enzyme of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.
In general the properties of the chosen enzyme(s) should be compatible with the selected detergent, (i.e. pH-optimum, compatibility with other enzymatic and non-enzymatic ingredients, etc.), and the enzyme(s) should be present in effective amounts.
Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Relase^{®}, Alcalase^{®}, Savinase^{®}, Primase^{®}, Everlase^{®}, Esperase^{®}, Ovozyme^{®}, Coronase^{®}, Polarzyme^{®} and Kannase^{®} (Novozymes A/S), Maxatase™, Maxacal™, Maxapem™, Properase™, Purafect™, Purafect OxP™, FN2™, FN3™, FN4™ and Purafect Prime™ (Genencor International, Inc.), BLAP X and BLAP S (Henkel).
Lipases: Suitable lipases include those of bacterial or fungal origin. Chemically modified or protein engineered mutants are included. Examples of useful lipases include lipases from *Humicola* (synonym *Thermomyces*), e.g. from *H. lanuginosa* (*T. lanuginosus*) as described in EP 258 068 and EP 305 216 or from *H. insolens* as described in WO 96/13580, a *Pseudomonas* lipase, e.g. from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202. Preferred commercially available lipase enzymes include Lipolase^{™} and Lipolase Ultra^{™} (Novozymes A/S).
Amylases: Suitable amylases (α and/or β) include those of bacterial or fungal origin. Chemically modified or protein engineered mutants are included. Amylases include, for example, α-amylases obtained from *Bacillus,* e.g. a special strain of *B. licheniformis*, described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444. Commercially used amylases are Duramyl^{®}, Termamyl^{®}, Stainzyme^{®}, Fungamyl^{®} and BAN^{®} (Novozymes A/S), Rapidase^{™}, Purastar^{™} and Purastar OxAM^{™} (from Genencor International Inc.).
Cellulases: Suitable cellulases include those of bacterial or fungal origin. Chemically modified or protein engineered mutants are included. Suitable cellulases include cellulases from the genera *Bacillus*, *Pseudomonas*, *Humicola*, *Fusarium*, *Thielavia*, *Acremonium*, e.g. the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, Renozyme^{®} and Carezyme™ (Novozymes A/S), Clazinase™, and Puradax HA™ (Genencor International Inc.), and KAC-500(B)™ (Kao Corporation).
Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g. from *C. cinereus,* and variants thereof as those described in WO 93/24618, WO 95/10602, and WO 98/15257.
Commercially available peroxidases include Guardzyme™ (Novozymes A/S).
Hemicellulases: Suitable hemicellulases include those of bacterial or fungal origin. Chemically modified or protein engineered mutants are included. Suitable hemicellulases include mannanase, lichenase, xylanase, arabinase, galactanase acetyl xylan esterase, glucorunidase, ferulic acid esterase, coumaric acid esterase and arabinofuranosidase as described in WO 95/35362. Suitable mannanases are described in WO 99/64619.

The detergent enzyme(s) may be included in a detergent composition by adding separate additives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste or a liquid. A liquid detergent may be aqueous, typically containing up to 70 % water and 0-30 % organic solvent, or non-aqueous.

The detergent composition comprises one or more surfactants, which may be non-ionic including semi-polar and/or anionic and/or cationic and/or zwitterionic. The surfactants are typically present at a level of from 0.1 % to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The detergent may contain 0-65 % of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system which may comprise a H₂O₂ source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

The detergent may also contain other conventional detergent ingredients such as e.g. fabric conditioners including clays, foam boosters, suds suppressors, anti-corrosion agents, soil-suspending agents, anti-soil redeposition agents, dyes, bactericides, optical brighteners, hydrotropes, tamish inhibitors, or perfumes.

In the detergent compositions any enzyme, in particular the enzyme of the invention, may be added in an amount corresponding to 0.01-100 mg of enzyme protein per litre of wash liquor, preferably 0.05-5 mg of enzyme protein per litre of wash liquor, in particular 0.1-1 mg of enzyme protein per litre of wash liquor.

The enzyme of the invention may additionally be incorporated in the detergent formulations disclosed in WO 97/07202 which is hereby incorporated as reference.

Typical powder detergent compositions for automated dishwashing include:
1)

| | |
|---|---|
| Nonionic surfactant | 0.4 -2.5% |
| Sodium metasilicate | 0 -20% |
| Sodium disilicate | 3 -20% |
| Sodium triphosphate | 20 - 40% |
| Sodium carbonate | 0 - 20% |
| Sodium perborate | 2 - 9% |
| Tetraacetyl ethylene diamine (TAED) | 1 - 4% |
| Sodium sulphate | 5 - 33% |
| Enzymes | 0.0001 - 0.1% |

2)

| | |
|---|---|
| Nonionic surfactant (e.g. alcohol ethoxylate) | 1 - 2% |
| Sodium disilicate | 2 - 30% |
| Sodium carbonate | 10 - 50% |
| Sodium phosphonate | 0 - 5% |
| Trisodium citrate dehydrate | 9 - 30% |
| Nitrilotrisodium acetate (NTA) | 0 - 20% |
| Sodium perborate monohydrate | 5 - 10% |
| Tetraacetyl ethylene diamine (TAED) | 1 - 2% |
| Polyacrylate polymer | |
| (e.g. maleic acid/acrylic acid copolymer) | 6 - 25% |
| Enzymes | 0.0001 - 0.1% |
| Perfume | 0.1 - 0.5% |
| Water | 5 -10 |

3)

| | |
|---|---|
| Nonionic surfactant | 0.5 - 2.0% |
| Sodium disilicate | 25 - 40% |
| Sodium citrate | 30 - 55% |
| Sodium carbonate | 0 - 29% |
| Sodium bicarbonate | 0 - 20% |
| Sodium perborate monohydrate | 0 - 15% |
| Tetraacetyl ethylene diamine (TAED) | 0 - 6% |
| Maleic acid/acrylic acid copolymer | 0 - 5% |
| Clay | 1 - 3% |
| Polyamino acids | 0 - 20% |
| Sodium polyacrylate | 0 - 8% |
| Enzymes | 0.0001 - 0.1% |

4)

| | |
|---|---|
| Nonionic surfactant | 1 - 2% |
| Zeolite MAP | 15 - 42% |
| Sodium disilicate | 30 - 34% |
| Sodium citrate | 0 - 12% |
| Sodium carbonate | 0 - 20% |
| Sodium perborate monohydrate | 7 - 15% |
| Tetraacetyl ethylene diamine (TAED) | 0 - 3% |
| Polymer | 0 - 4% |
| Maleic acid/acrylic acid copolymer | 0 - 5% |
| Organic phosphonate | 0 - 4% |
| Clay | 1 - 2% |
| Enzymes | 0.0001 - 0.1% |
| Sodium sulphate | Balance |

5)

| | |
|---|---|
| Nonionic surfactant | 1 - 7% |
| Sodium disilicate | 18 - 30% |
| Trisodium citrate | 10 - 24% |
| Sodium carbonate | 12 - 20% |
| Monopersulphate (2 KHSO₅.KHSO₄.K₂SO₄) | 15 -21% |
| Bleach stabilizer | 0.1 - 2% |
| Maleic acid/acrylic acid copolymer | 0 - 6% |
| Diethylene triamine pentaacetate, pentasodium salt | 0 - 2.5% |
| Enzymes | 0.0001 - 0.1% |
| Sodium sulphate, water | Balance |

Powder and liquid dishwashing compositions with cleaning surfactant system typically include the following ingredients:
6)

| | |
|---|---|
| Nonionic surfactant | 0 - 1.5% |
| Octadecyl dimethylamine N-oxide dihydrate | 0 - 5% |
| 80:20 wt.C18/C16 blend of octadecyl dimethylamine | |
| N-oxide dihydrate and hexadecyldimethyl amine N-oxide dihydrate | 0 - 4% |
| 70:30 wt.C18/C16 blend of octadecyl bis (hydroxylethyl)amine N-oxide anhydrous and hexadecyl bis (hydroxyethyl)amine N-oxide anhydrous | 0 - 5% |
| C₁₃-C₁₅ alkyl ethoxysulfate with an average degree of ethoxylation of 3 | 0 - 10% |
| C₁₂-C₁₅ alkyl ethoxysulfate with an average degree of ethoxylation of 3 | 0 - 5% |
| C₁₃-C₁₅ ethoxylated alcohol with an average degree of ethoxylation of 12 | 0 - 5% |
| A blend of C₁₂-C₁₅ ethoxylated alcohols with an average degree of ethoxylation of 9 | 0 - 6.5% |
| A blend of C₁₃-C₁₅ ethoxylated alcohols with an average degree of ethoxylation of 30 | 0 - 4% |
| Sodium disilicate | 0 - 33% |
| Sodium tripolyphosphate | 0 - 46% |
| Sodium citrate | 0 - 28% |
| Citric acid | 0 - 29% |
| Sodium carbonate | 0 - 20% |
| Sodium perborate monohydrate | 0 - 11.5% |
| Tetraacetyl ethylene diamine (TAED) | 0 - 4% |
| Maleic acid/acrylic acid copolymer | 0 - 7.5% |
| Sodium sulphate | 0 - 12.5% |
| Enzymes | 0.0001 - 0.1% |

Non-aqueous liquid ADW compositions typically include the following ingredients:
7)

| | |
|---|---|
| Liquid nonionic surfactant e.g. alcohol ethoxylates | 2.0 -10.0% |
| Alkali metal silicate | 3.0 - 15.0% |
| Alkali metal phosphate | 20.0 - 40.0% |
| Liquid carrier selected from higher glycols, polyglycols, polyoxides, glycolethers | 25.0 - 45.0% |
| Stabilizer (e.g. a partial ester of phosphoric acid and a C₁₆-C₁₈ alkanol) | 0.5 - 7.0% |
| Foam suppressor (e.g. silicone) | 0 - 1.5% |
| Enzymes | 0.0001 - 0.1% |

8)

| | |
|---|---|
| Liquid nonionic surfactant e.g. alcohol ethoxylates | 2.0 - 10.0% |
| Sodium silicate | 3.0 - 15.0% |
| Alkali metal carbonate | 7.0 - 20.0% |
| Sodium citrate | 0.0 - 1.5% |
| Stabilizing system (e.g. mixtures of finely divided silicone and low molecular weight dialkyl polyglycol ethers) | 0.5 - 7.0% |
| Low molecule weight polyacrylate polymer | 5.0 - 15.0% |
| Clay gel thickener (e.g. bentonite) | 0.0 - 10.0% |
| Hydroxypropyl cellulose polymer | 0.0 - 0.6% |
| Enzymes | 0.0001 - 0.1% |
| Liquid carrier selected from higher lycols, polyglycols, polyoxides and glycol ethers | Balance |

Thixotropic liquid ADW compositions typically include the following ingredients:
9)

| | |
|---|---|
| C₁₂-C₁₄ fatty acid | 0 - 0.5% |
| Block co-polymer surfactant | 1.5 - 15.0% |
| Sodium citrate | 0 - 12% |
| Sodium tripolyphosphate | 0 - 15% |
| Sodium carbonate | 0 - 8% |
| Aluminium tristearate | 0 - 0.1% |
| Sodium cumene sulphonate | 0 - 1.7% |
| Polyacrylate thickener | 1.32 - 2.5% |
| Sodium polyacrylate | 2.4 - 6.0% |
| Boric acid | 0 - 4.0% |
| Sodium formate | 0 - 0.45% |
| Calcium formate | 0 - 0.2% |
| Sodium n-decydiphenyl oxide disulphonate | 0 - 4.0% |
| Monoethanol amine (MEA) | 0 - 1.86% |
| Sodium hydroxide (50%) | 1.9 - 9.3% |
| 1,2-Propanediol | 0 - 9.4% |
| Enzymes | 0.0001 - 0.1% |
| Suds suppressor, dye, perfumes, water | Balance |

Liquid automatic dishwashing compositions typically include the following ingredients:
10)

| | |
|---|---|
| Alcohol ethoxylate | 0 - 20% |
| Fatty acid ester sulphonate | 0 - 30% |
| Sodium dodecyl sulphate | 0 - 20% |
| Alkyl polyglycoside | 0 - 21% |
| Oleic acid | 0 - 10% |
| Sodium disilicate monohydrate | 18 - 33% |
| Sodium citrate dihydrate | 18 - 33% |
| Sodium stearate | 0 - 2.5% |
| Sodium perborate monohydrate | 0 - 13% |
| Tetraacetyl ethylene diamine (TAED) | 0 - 8% |
| Maleic acid/acrylic acid copolymer | 4 - 8% |
| Enzymes | 0.0001 - 0.1% |

Liquid ADW compositions containing protected bleach particles typically include the following ingredients:
11)

| | |
|---|---|
| Sodium silicate | 5 - 10% |
| Tetrapotassium pyrophosphate | 15 - 25% |
| Sodium triphosphate | 0 - 2% |
| Potassium carbonate | 4 - 8% |
| Protected bleach particles, e.g. chlorine | 5 - 10% |
| Polymeric thickener | 0.7 - 1.5% |
| Potassium hydroxide | 0 - 2% |
| Enzymes | 0.0001 - 0.1% |
| Water | Balance |

12) Automatic dishwashing compositions as described in 1), 2), 3), 4), 6) and 10), wherein perborate is replaced by percarbonate.
13) Automatic dishwashing compositions as described in 1) - 6) which additionally contain a manganese catalyst. The manganese catalyst may, e.g., be one of the compounds described in "Efficient manganese catalysts for low-temperature bleaching", Nature 369, 1994, pp. 637-639.

### MATERIALS AND METHODS

### Method for producing a subtilase variant

The present invention provides a method of producing an isolated enzyme according to the invention, wherein a suitable host cell, which has been transformed with a DNA sequence encoding the enzyme, is cultured under conditions permitting the production of the enzyme, and the resulting enzyme is recovered from the culture.

When an expression vector comprising a DNA sequence encoding the enzyme is transformed into a heterologous host cell it is possible to enable heterologous recombinant production of the enzyme of the invention. Thereby it is possible to make a highly purified subtilase composition, characterized in being free from homologous impurities.

The medium used to culture the transformed host cells may be any conventional medium suitable for growing the host cells in question. The expressed subtilase may conveniently be secreted into the culture medium and may be recovered there-from by well-known procedures including separating the cells from the medium by centrifugation or filtration, precipitating proteinaceous components of the medium by means of a salt such as ammonium sulfate, followed by chromatographic procedures such as ion exchange chromatography, affinity chromatography, or the like.

### Example 1

### Selection of strains and screening with antibodies

In the search for Bacillus strains producing novel subtilases of the PD138 group we selected a number of strains, which based on 16S rDNA similarity was related to *Bacillus gibsonii.* A number of such Bacillus strains were fermented in a standard Bacillus fermentation medium (BP-X added 0.1 M NaHCO3 to adjust pH to 9).

The immunochemical properties can be determined immunologically by cross-reaction identity tests. The identity test can be performed either by the well known ouchterlony double immuno diffusion procedure or by tandem crossed immunoelectro-phoresis according to N.H Axelsen, Handbook of immunoprecipitation-in-gel Techniques. Blackwell Scientific Publications (1983) chapters 5 &14.

Culture fluids were analysed for protease activity using Alcalase^{™} as standard. Fluids with 10 CPU/L or more activity was included in the immunological analysis.

The analysis included two different antibodies; AB41 is a polyclonal rabbit antibody raised against the PD138 protease (WO 93/18140). The other antibody is AB65 raised against a bacterial subtilisin isolated from wild type *Bacillus sp.* PD490 (not published). The analysis revealed two novel groups of proteases with a partial reaction against the AB41. One of these groups also had a partial reaction against AB65 (EP655, ZI120, EP63, ZI130 and ZI132), whereas the other group reacted identical with AB65 (ZI344 and ZI430). A third group including the PD138 protease reacted identical with AB41 and partially identical with AB65.

**Table 1. Different proteases and their reaction with two different antibodies**

| Protease \ Antibody | AB41 | AB65 |
|---|---|---|
| PD138 | Identical | Partial |
| EP655 | Partial | Partial |
| ZI120 | Partial | Partial |
| EP63 | Partial | Partial |
| ZI130 | Partial | Partial |
| ZI132 | Partial | Partial |
| ZI344 | Partial | Identical |
| ZI340 | Partial | Identical |

A part of the subtilase gene was amplified with a standard PCR reaction with PCR primers: PD138AO (SEQ ID NO:7)/ PD138A2 (SEQ ID NO:9) gave a PCR product of about 900 nt; PD138A1 (SEQ ID NO:8)/ PD138A2 (SEQ ID NO:9) gave a PCR product of about 450 nt; ZI344F (SEQ ID NO:10)/ PD138A2 (SEQ ID NO:9) gave a PCR product of about 800 nt.
GAGGAGGCNGAGTTNGARGC (SEQ ID NO:7), the symbols for degenerations are: N for inosine and R for an equal mixture of A and G.
AGTTAGCAGATATAAATAATTCAA (SEQ ID NO:8),
GTGGAGTAGCCATAGATGTACCA (SEQ ID NO:9),
TGCAAACGAGGTTGAACAGG (SEQ ID NO:10).
The PCR reaction that included 50U/ml of Ampli-taq™ DNA polymerase (Perkin Elmer) 10x Amplitaq buffer (final concentration of MgCl₂ is 1.5 mM) 0.2 mM of each of the dNTPs (dATP, dCTP, dTTP and dGTP), 0.2 pmol/µl of the primers and 1 µl DNA template.

Template DNA was recovered from the various Bacillus strains using HighPure™ PCR template preparation kit (Boehringer Mannheim art. 1796828) as recommended by the manufacturer for DNA recovery from bacteria. The quality of the isolated template was evaluated by agarose gel electrophoresis. If a high molecular weight band was present the quality was accepted. PCR was run in the following protocol: 94°C, 2 minutes 40 cycles of [94°C for 30 seconds, 52°C for 30 seconds, 68°C for 1 minute] completed with 68°C for 10 minutes. PCR products were analysed on a 1% agarose gel in TAE buffer stained with Ethidium bromide to confirm a single band of app. 1050 nucleotides. The PCR product was recovered by using Qiagen™ PCR purification kit as recommended by the manufacturer. The nucleotide sequences were determined by sequencing on an ABI PRISM™ DNA sequencer (Perkin Elmer).

The nucleotide sequences were analysed with DNA STAR^{™}, and based on nucleotide sequence diversity with PD138 as benchmark the novel groups identified with antibodies were confirmed. A phylogenetic tree based on the sequences from the PCR screening is presented in Figure 1. A ClustalV alignment of the sequences from the PCR screening is shown in Figure 2.

### EXAMPLE 2 Production of full length subtilases

### Inverse PCR

Three digestions of the chromosomal DNA of the strains EP655, P203 and ZI344 were made using the restriction enzymes Mlu1, EcoR1 and Sac1. Upon digestion the DNA was separated from the restriction enzymes using Qiaquick™ PCR purification kit (art. 28106, Qiagen, Germany). The digestions were religated and subjected to a PCR reaction using primers (PCR primers SEQ ID NO's:11-16) designed to recognise the sequence of the PCR product already obtained. The following PCR protocols were applied: 94°C 2 min 30 cycles of [94°C for 15 s, 52°C for 30 s, 72°C for 2 min] 72°C 20 min. in the PCR the amount of primer, DNA polymerase and buffer were the same as in Example 1. Alternatively a protocol with 94°C 2 min 30 cycles of [94°C for 15 s, 52°C for 30 s, 68°C for 3 min] 68°C 20 min. and replacing Amplitaq^{®} and Amplitaq^{®} buffer with Long-template Taq polymerase^{™} (Boehringer Mannheim) with the buffer supplied with the polymerase. The PCR reactions were analysed on 0.8% agarose gels stained with ethidium bromide. All PCR fragments were recovered and the nucleotide sequence was determined by using specific oligo primers different from those used in the PCR reaction (Sequencing primers SEQ ID NO's:17-22).
The following primers were used for obtaining the inverse PCR and sequencing:
Inverse PCR primers
P203A-PCR-R (SEQ ID NO:11) ACACGAGTAATACCCCAAGG
P203A-PCR-F (SEQ ID NO:12) GCTAATGCAATGGCAGTAGG
ZI344-PCR-R (SEQ ID NO:13) ACTCTTTGAATGCCCCAAGG
ZI344-PCR-F (SEQ ID NO:14) AGGTGTACTTGTTGTGGCAG
EP655-PCR-R (SEQ ID NO:15) AGTAATACCCCAAGGCACCG
EP655-PCR-F (SEQ ID NO:16) GCGGCTTCAGGTAATAACGG

### Sequencing primers

P203A-seq-R (SEQ ID NO:17) CAACTCAACTGATAATACGG
P203A-seq-F (SEQ ID NO:18) TTCTCTCAATATGGTGCAGG
EP655-seq-R (SEQ ID NO:19) AATGCATCAACATCTTCAGG
EP655-seq-F (SEQ ID NO:20) GGATATCCTGCACGTTATGC
ZI344-seq-R (SEQ ID NO:21) AGTGCTTCTACATCCTCAGG
ZI344-seq-F (SEQ ID NO:22) AACGTTGGCTACCCTGCACG

### Production of the full length subtilase

To produce the subtilases of strains P203, EP655 and ZI344 the protease gene was amplified from chromosomal DNA of the wild type strains. For P203 chromosomal DNA of the strain DSM 17419 can be used. The protease gene was amplified as a app. 1200 nt (nucleotide) PCR product. For P203 primers P203A-Sac1/P203A-BamH1 for Zi344 primers ZI344-Sac1/ZI344-Mlu1 and for EP655 primers P203A-Sac1/EP655-MLu1 were used. Template DNA was chromosomal DNA of the respective wild type Bacillus strains.
Primers:
P203A-Sac1: TTATGGAGCTCCTAAAAATGAGGAGGCGACC (SEQ ID NO. 23)
P203A-BamH1: TGTATGGATCCAAATAGAGACGAAACCGCCC (SEQ ID NO. 24)
EP655-MLu1: GATTAACGCGTCTGCTCTTATCGACTAGCGG (SEQ ID NO. 25)
ZI344-Sac1: TTATGGAGCTCGATCAATACAAGGAGGCGAC (SEQ ID NO. 26)
ZI344-Mlu1: GATTAACGCGTGTTCTTTTATCGTGTAGCTG (SEQ ID NO. 27)
EP655-Sac1: use P203A-Sac1.

The PCR products were recovered using Qiaquick^{tm} spin columns as recommended (Qiagen, Germany). The quality of the isolated template was evaluated by agarose gel electrophoresis. PCR was run in the following protocol: 94°C, 2 minutes 40 cycles of [94°C for 30 seconds, 52°C for 30 seconds, 68°C for 1 minute] completed with 68°C for 10 minutes. PCR products were analysed on a 1% agarose gel in TAE buffer stained with Ethidium bromide to confirm a single band of the correct size. The PCR products were digested with restriction enzymes Sac1 and Mlu1 and purified on GFX^{™} PCR and Gel Band Purification Kit (Amerham Biosciences).

The digested and purified PCR fragment was ligated to the Sac I and Mlu I digested plasmid pDG268NeoMCS-PramyQ/PrcryIII/cryIIIAstab/Sav (US 5,955,310). The ligation mixture was used for transformation into *E*. *coli* TOP10F' (Invitrogen BV, The Netherlands) and several colonies were selected for miniprep (QIAprep^{®} spin, QIAGEN GmbH, Germany). The purified plasmids were checked for insert before transformation into a strain of *Bacillus subtilis* derived from *B. subtilis* DN 1885 with disrupted apr, npr and pel genes (Diderichsen et al (1990), J. Bacteriol., 172, 4315-4321). The disruption was performed essentially as described in "Bacillus subtilis and other Gram-Positive Bacteria," American Society for Microbiology, p. 618, eds. A.L. Sonenshein, J.A. Hoch and Richard Losick (1993). Transformed cells were plated on 1% skim milk LB-PG agar plates, supplemented with 6 µg/ml chloramphenicol. The plated cells were incubated over night at 37°C and protease containing colonies were identified by a surrounding clearing zone. Protease positive colonies were selected and the coding sequence of the expressed enzyme from the expression construct was confirmed by DNA sequence analysis.

### EXAMPLE 3 Purification and characterisation

### Purification

This procedure relates to purification of a 2 liter scale fermentation for the production of the subtilases of the invention in a *Bacillus* host cell.
Approximately 1.6 liters of fermentation broth are centrifuged at 5000 rpm for 35 minutes in 1 liter beakers. The supernatants are adjusted to pH 6.5 using 10% acetic acid and filtered on Seitz Supra^{®} S100 filter plates.

The filtrates are concentrated to approximately 400 ml using an Amicon^{®} CH2A UF unit equipped with an Amicon^{®} S1Y10 UF cartridge. The UF concentrate is centrifuged and filtered prior to absorption at room temperature on a Bacitracin affinity column at pH 7. The protease is eluted from the Bacitracin column at room temperature using 25% 2-propanol and 1 M sodium chloride in a buffer solution with 0.01 dimethylglutaric acid, 0.1 M boric acid and 0.002 M calcium chloride adjusted to pH 7.

The fractions with protease activity from the Bacitracin purification step are combined and applied to a 750 ml Sephadex^{®} G25 column (5 cm dia.) equilibrated with a buffer containing 0.01 dimethylglutaric acid, 0.2 M boric acid and 0.002 m calcium chloride adjusted to pH 6.5.

Fractions with proteolytic activity from the Sephadex^{®} G25 column are combined and applied to a 150 ml CM Sepharose^{®} CL 6B cation exchange column (5 cm dia.) equilibrated with a buffer containing 0.01 M dimethylglutaric acid, 0.2 M boric acid, and 0.002 M calcium chloride adjusted to pH 6.5.
The protease is eluted using a linear gradient of 0-0.1 M sodium chloride in 2 litres of the same buffer.

In a final purification step subtilase containing fractions from the CM Sepharose^{®} column are combined and concentrated in an Amicon^{®} ultrafiltration cell equipped with a GR81PP membrane (from the Danish Sugar Factories Inc.).

### EXAMPLE 4 Stability of subtilases

The stability of the subtilases of the invention can be evaluated in a standard Western European dishwashing tablet detergent without other enzymes than the experimentally added subtilases. The stability of the subtilases can be determined as the residual proteolytic activity after incubation of the subtilase in a detergent.
The formulation of a standard Western European Tablet detergent is defined as:

| **Component** | **Percentage** |
|---|---|
| Non-ionic surfactants | 0-10% |
| Foam regulators | 1-10% |
| Bleach (per-carbonate or per-borate) | 5-15% |
| Bleach activators (e.g. TAED) | 1-5% |
| Builders (e.g. carbonate, phosphate, tri-phosphate, Zeolite) | 50-75% |
| Polymers | 0-15% |
| Perfume, dye etc. | <1% |
| Water and fillers (e.q. sodium sulphate) | Balance |

### Assay for Proteolytic Activity

The proteolytic activity is determined with casein as substrate. One Casein Protease Unit (CPU) is defined as the amount of protease liberating about 1 µM of primary amino groups (determined by comparison with a serine standard) per minute under standard conditions, i.e., incubation for about 30 minutes at about 25°C at pH 9.5.
The proteolytic activity may also be determined by measuring the specific hydrolysis of succinyl-Ala-Ala-Pro-Leu-p-nitroanilide by said protease. The substrate is initially dissolved in for example, DMSO (Dimethyl Sulfoxide) and then diluted about 50 fold in about 0.035 M borate buffer, about pH 9.45. All protease samples may be diluted about 5-10 fold by the same borate buffer. Equal volumes of the substrate solution and sample are mixed in a well of an ELISA reader plate and read at about 405 nm at 25°C. All sample activities and concentrations are normalized to the standard protease solution activity and concentration, respectively.

A typical Western European tablet detergent for automated dishwashing is dissolved (5.5 g/L) in 9°dH water at ambient temperature maximum 30 minutes prior to start of analyses. Samples of subtilases are diluted to a concentration of 2-4 CPU/ml in Britten Robinson buffer (Britten Robinson buffer is: 40 mM Phosphate, 40 mM Acetate and 40 mM Borate) pH9.5. For the analyses every sample is divided and tested under two conditions: For the control the subtilase is diluted 1:9 in Britten Robinson buffer pH9.5 to a final volume of 1 ml. This sample is analysed immediately after dilution. For the detergent stability the subtilase sample is diluted 1:9 in detergent solution (detergent concentration in the stability test is 5 g/L) these samples are incubated at 55°C for 30 minutes prior to analysis by addition of casein substrate.

The assay is started by addition of 2 volumes of casein substrate (casein substrate is 2 g of casein (Merck, Hammerstein grade) in 100 ml of Britten Robinson buffer pH 9.5, pH is re-adjusted to 9.5 when the casein is in solution). Samples are kept isothermic at 25°C for 30 minutes. The reaction is stopped by addition of 5 ml TCA solution (TCA solution is 89.46 g of Tri-chloric acid, 149.48 g of Sodium acetate-tri-hydrate and 94.5 ml of glacial acetic acid in 2.5 L of deionised water). The samples are incubated at ambient temperature for at least 20 minutes and filtered through Whatman^{®} paper filter no. 42.

400µl of filtrate is mixed with 3 ml OPA reagent (OPA reagent is composed of: 3.812 g of borax, 0.08% EtOH, 0.2% DTT and 80 mg of o-phthal-dialdehyd in 100 ml water). Absorption at 340nm is measured and CPU is calculated from the concentration of free amines on a standard of a solution of 0.01% L-serine (Merck art. 7769).

### EXAMPLE 5 Microtiter egg assay (MEA)

In this assay the digestion of denatured egg proteins by proteases in the presence of detergent can be followed in a 96-well microtiter plate. Heating of egg proteins produces visual changes and changes in physicochemical properties. The clear translucent material is transformed to a milky substance. This is partly due to sulfhydryl-disulfide interchange reactions of denatured proteins. For example, heating unmasks the sulfhydryl group of ovalbumin, and the unmasked groups form disulfide linkages. The digestion of the denatured egg proteins by proteases converts the milky egg solution to a more clear solution dependent on the ability of the enzymes to degrade egg proteins.

### Procedure

a) Prepare an egg solution by dissolving 200mg egg powder (Sanovo International AS) in 93,7 mL, where the water hardness in adjusted to 16°dH. Denature the egg solution by increasing the temperature to 85° C over an 8 minutes time period.
b) Dilute the subtilase enzyme to 320nM in succinic acid buffer: 10mM succinic acid + 2 mM CaCl₂+ 0,02% non-ionic detergent (such as Brij35) adjusted to pH 6,5;
c) Prepare the detergent solution just before use by mixing 5g detergent & 937.5mL water (16 °dH(Ca²⁺/Mg²⁺ 4:1)). The dishwash detergent could be a typical Western Europe 2in1 (use 8°dH) or 3in1 tablet (use 16°dH) or an automatic dishwash powder product (use 8°dH). If the detergent already contains proteases, the detergent solution should be inactivated in a microwave oven at 85°C for 5 minutes
d) Add to each well in a 96 well microtiter plate: 10µl of 320nM enzyme solution (final concentration 20nM) + 150µl detergent solution (final concentration 5g/L, 16°d) + egg solution (320µg egg protein/well).

Measure OD 410nm immediately (time 0 minutes) on a spectrophotometer. Incubate exactly 20 minutes at 55°C and then measure OD 410nm again. Calculate ΔOD and compare the variants with the performance of a reference subtilase, such as Savinase^{®} or Alcalase^{®} from Novozymes A/S. The performance of the reference is set to ΔOD = 100%.

By use of the above mentioned procedure the digestion of denatured egg proteins by the subtilase enzymes of the invention was compared with that of Savinase^{®}. The results are presented in Table 1 as performance % of Savinase performance:

**Table 1**

| Savinase | Alcalase | EP655 | ZI344 | P203 |
|---|---|---|---|---|
| 100 | 10 | 211 | 230 | 212 |

## Claims

1. An isolated polypeptide having subtilase activity which polypeptide has an amino acid sequence which is:
a) as shown in SEQ ID NO:2;
b) at least 90% identical with the sequence of SEQ ID NO:2;.

2. A nucleic acid sequence encoding a polypeptide having subtilase activity, which sequence is:
a) as shown in SEQ ID NO:1;
b) at least 81% identical with the sequence shown in SEQ ID NO:1.

3. A nucleic acid construct comprising the nucleic acid sequence of claim 2 operably linked to one or more control sequences capable of directing the expression of the polypeptide in a suitable expression host.

4. A recombinant expression vector comprising the nucleic acid construct of claim 3, a promoter, and transcriptional and translational stop signals.

5. The vector according to claim 4, further comprising a selectable marker.

6. A recombinant host cell comprising the nucleic acid construct of claim 3.

7. The cell according to claim 6, wherein the nucleic acid construct is contained on a vector.

8. The cell according to claim 7, wherein the nucleic acid construct is integrated into the host cell genome.

9. The cell according to claim 8, wherein the nucleic acid sequence encodes an amino acid sequence set forth in SEQ ID NO:2.

10. The cell according to claim 9, wherein the nucleic acid sequence is set forth in SEQ ID NO: 1.

11. A method for producing the polypeptide of claim 1 comprising (a) cultivating a host cell according to claim 6 comprising a nucleic acid construct comprising a nucleic acid sequence encoding the polypeptide under conditions conducive to expression of the polypeptide; and (b) recovering the polypeptide.

12. Use of a polypeptide having subtilase activity according to claim 1 in a detergent.

13. A detergent composition comprising a polypeptide having subtilase activity according to claim 1.

14. The detergent composition of claim 13, which is a laundry detergent or an automatic dishwashing detergent.

## Patentansprüche

1. Isoliertes Polypeptid mit Subtilaseaktivität, wobei das Polypeptid eine Aminosäuresequenz aufweist, die:
a) wie in SEQ ID NO: 2 gezeigt ist;
b) mindestens zu 90% mit der Sequenz von SEQ ID NO: 2 identisch ist.

2. Nukleinsäuresequenz, kodierend ein Polypeptid mit Subtilaseaktivität, wobei die Sequenz:
a) wie in SEQ ID NO: 1 gezeigt ist;
b) mindestens zu 81% mit der Sequenz von SEQ ID NO: 1 identisch ist.

3. Nukleinsäurekonstrukt, umfassend die Nukleinsäuresequenz gemäß Anspruch 2, funktionsfähig verknüpft mit einer oder mehreren Kontrollsequenz(en), die zum Steuern der Expression des Polypeptids in einem geeigneten Expressionswirt fähig ist/sind.

4. Rekombinanter Expressionsvektor, umfassend das Nukleinsäurekonstrukt gemäß Anspruch 3, einen Promotor und transkriptionale und translationale Stoppsignale.

5. Vektor nach Anspruch 4, des Weiteren umfassend einen Selektionsmarker.

6. Rekombinante Wirtszelle, umfassend das Nukleinsäurekonstrukt gemäß Anspruch 3.

7. Zelle nach Anspruch 6, wobei das Nukleinsäurekonstrukt auf einem Vektor enthalten ist.

8. Zelle nach Anspruch 7, wobei das Nukleinsäurekonstrukt in dem Wirtszellgenom integriert ist.

9. Zelle nach Anspruch 8, wobei die Nukleinsäuresequenz eine in SEQ ID NO: 2 dargelegte Aminosäuresequenz kodiert.

10. Zelle nach Anspruch 9, wobei die Nukleinsäuresequenz in SEQ ID NO: 1 dargelegt ist.

11. Verfahren zum Herstellen des Polypeptids gemäß Anspruch 1, umfassend (a) Kultivieren einer Wirtszelle gemäß Anspruch 6, umfassend ein Nukleinsäurekonstrukt, das eine das Polypeptid kodierende Nukleinsäuresequenz umfasst, unter Bedingungen, die für die Expression des Polypeptids förderlich sind; und (b) Gewinnen des Polypeptids.

12. Verwendung eines Polypeptids mit Subtilaseaktivität gemäß Anspruch 1 in einem Detergens.

13. Detergenszusammensetzung, umfassend ein Polypeptid mit Subtilaseaktivität gemäß Anspruch 1.

14. Detergenszusammensetzung nach Anspruch 13, das ein Wäschedetergens oder ein Detergens zum maschinellen Geschirrspülen ist.

## Revendications

1. Polypeptide isolé ayant une activité subtilase, lequel polypeptide a une séquence d'acides aminés qui est:
a) telle que montrée dans SEQ ID NO: 2;
b) au moins à 90% identique à la séquence de SEQ ID NO: 2.

2. Séquence d'acide nucléique codant un polypeptide ayant une activité subtilase, laquelle séquence est:
a) telle que montrée dans SEQ ID NO: 1;
b) au moins à 81% identique à la séquence montrée dans SEQ ID NO: 1.

3. Construction d'acide nucléique comprenant la séquence d'acide nucléique de la revendication 2 liée de manière fonctionnelle à une ou plusieurs séquences de contrôle capables d'orienter l'expression du polypeptide dans un hôte d'expression approprié.

4. Vecteur d'expression recombinant comprenant la construction d'acide nucléique de la revendication 3, un promoteur, et des signaux terminateurs de transcription et de traduction.

5. Vecteur selon la revendication 4, comprenant également un marqueur sélectionnable.

6. Cellule hôte recombinante comprenant la construction d'acide nucléique de la revendication 3.

7. Cellule selon la revendication 6, dans laquelle la construction d'acide nucléique est contenue dans un vecteur.

8. Cellule selon la revendication 7, dans laquelle la construction d'acide nucléique est intégrée dans le génome de la cellule hôte.

9. Cellule selon la revendication 8, dans laquelle la séquence d'acide nucléique code pour une séquence d'acides aminés présentée dans SEQ ID NO: 2.

10. Cellule selon la revendication 9, dans laquelle la séquence d'acide nucléique est présentée dans SEQ ID NO: 1.

11. Méthode pour produire le polypeptide de la revendication 1 comprenant (a) la culture d'une cellule hôte selon la revendication 6 comprenant une construction d'acide nucléique comprenant une séquence d'acide nucléique codant pour le polypeptide dans des conditions favorables à l'expression du polypeptide; et (b) la récupération du polypeptide.

12. Utilisation d'un polypeptide ayant une activité subtilase selon la revendication 1 dans un détergent.

13. Composition détergente comprenant un polypeptide ayant une activité subtilase selon la revendication 1.

14. Composition détergente selon la revendication 13, laquelle est un détergent de blanchisserie ou un détergent de laverie automatique.
